# EUROPEAN PATENT SPECIFICATION

(11) **EP 3 738 501 B1**
(45) Date of publication and mention of the grant of the patent: **22.05.2024**
(21) Application number: 18899048.5
(22) Date of filing: 27.12.2018
(51) Int. Cl.: A61B 3/14, A61B 3/15

(54) **FUNDUS IMAGING DEVICE**
AUGENHINTERGRUNDABBILDUNGSVORRICHTUNG
DISPOSITIF D'IMAGERIE DE FOND D'OEIL

(30) Priority: 10.01.2018 JP 2018002250; 10.01.2018 JP 2018002249
(43) Date of publication of application: 18.11.2020
(73) Proprietor: Nidek Co., Ltd., Gamagori-shi, Aichi, 443-0038 (JP)
(72) Inventor: YOSHINO, Masayuki, Gamagori-shi, Aichi 443-0038 (JP); IWATA, Shinya, Gamagori-shi, Aichi 443-0038 (JP)
(74) Representative: Hoefer & Partner Patentanwälte mbB
(86) International application number: PCT/JP2018/048339
(87) International publication number: WO 2019/138916

(56) References cited:
- JP-A- H11 253 403
- JP-A- S61 276 534
- JP-A- 2002 051 985
- JP-A- 2005 087 546
- JP-A- 2005 160 549
- JP-A- 2005 312 751
- JP-A- 2005 529 669
- JP-A- 2012 213 632
- JP-A- 2014 113 385
- JP-A- 2015 195 808
- JP-A- 2015 195 874
- JP-A- 2016 030 181
- JP-A- 2016 049 368
- JP-A- 2016 185 192
- JP-B2- S6 148 940
- US-A1- 2006 177 205

## Description

### Technical Field

The present disclosure relates to a fundus imaging device for obtaining a front image of a fundus.

### Background Art

A fundus imaging device that captures a front image of a fundus of a subject eye is widely used in an ophthalmic field.

For example, a device that obtains a front image of a fundus by scanning slit-shaped illumination light on the fundus and sequentially projecting an image of an illuminated fundus region on a two-dimensional imaging surface according to the scanning is known as a kind of a fundus imaging device. As a device of this type, Patent Literature 1 discloses a device that performs imaging by disposing two hole apertures in which two openings are formed in a scan direction of a slit at a surface conjugating with a pupil of a light projection system, simultaneously emitting illumination light from two openings of two hole apertures, and extracting fundus reflection light from a gap between the two hole apertures projected on a pupil.

JP-A-2005/160 549 discloses a fundus camera with a photographing optical system having a focusing lens movable in an optical axis direction for photographing a fundus oculi of an eye to be examined, which is illuminated by a photographing illumination light, an alignment detecting means detecting the alignment state of the photographing optical system relative to the eye to be examined, a focus detecting means detecting the focus state of the fundus oculi adjusted by the movement of the focusing leans, a pupil and visual line detecting means detecting at least one of the pupil state and the visual line direction of the eye to be examined, and an automatic photographing means automatically executing the photographing by the photographing optical system based on the detection result of the detecting means.

### Citation List

### Patent Literature

Patent Literature 1: JP-A-S61-48940

### Summary of Invention

However, in the device of Patent Literature 1, in a case where a pupil diameter of a subject eye is small, it is considered a case where images of two openings of the two hole apertures are not disposed inside the pupil of the subject eye, the illumination light is vignetted by an iris, and thereby, a fundus image becomes dark.

The present disclosure is made in view of the problems of the related art, and an object of the present disclosure is to provide a fundus imaging device that easily images a subject eye having a smaller pupil diameter. This object is solved by the features of the independent claim. The dependent claims contain advantageous embodiments of the present invention.

A fundus imaging device according to the invention is defined in claim 1.

The fundus imaging device may further include an anterior eye segment observation optical system that obtains an anterior eye segment observation image, and the imaging control means sets the alignment reference position based on information regarding a pupil region of the subject eye acquired from the anterior eye segment observation image.

The information regarding the pupil region may include a ratio of a part, which is disposed inside the pupil region, of the light projection region and the light receiving region.

The fundus imaging device may include observation image obtaining means that obtains a fundus observation image of the fundus image, and the imaging control means sets the alignment reference position based on information regarding brightness of the fundus observation image.

The fundus imaging device may include an anterior eye segment observation optical system that obtains an anterior eye segment observation image of the subject eye; and operation input means that receives an operation of an examiner and drives the drive unit according to the operation to adjust the relative position, in which the imaging control means includes display control means that displays the anterior eye segment observation image on a monitor and displays a guide, which guides an operation to a target position, on the anterior eye segment observation image based on the alignment reference position.

The imaging control means may drive and control the drive unit based on an alignment deviation from the alignment reference position to induce alignment.

The light projection and reception separating means may form two light projection regions having different positions on a pupil of the subject eye, and the imaging control means may set a position, as the second reference position, at which one of the two light projection regions together with the light receiving region are disposed inside a pupil of the subject eye more preferentially than a remaining one light projection region.

The remaining one of the two light projection regions is disposed outside the pupil at the second reference position.

A center of the light receiving region may match a center of the pupil or a center of a cornea of the subject eye at the first reference position.

In a case of inducing alignment to the second reference position, the imaging control means may increase at least one of an amount of the illumination light and a gain of the imaging element to capture the fundus image, as compared with a case of inducing alignment to the first reference position.

The imaging control means may cause the illumination light to be projected to a fundus from both of the two light projection regions when the fundus image is captured in a case of inducing alignment to the first reference position, and the illumination light to be projected to the fundus from only one of the two light projection regions, which is preferentially disposed inside the pupil when the fundus image is captured in a case of inducing alignment to the second reference position.

The light projection and reception separating means may form the two light projection regions and the light receiving region at positions separated in a left-right direction.

The light projection and reception separating means may form the two light projection regions at positions vertically separated from each other, and the imaging control means may set the second reference position at which one of the two light projection regions formed on a lower side is disposed inside the pupil, and projects the illumination light from the one when capturing the fundus image.

The light projection and reception separating means may form the light receiving region to be interposed between the two light projection regions on the pupil of the subject eye.

The imaging optical system may further include a slit formation unit that forms the illumination light in a slit shape on the fundus of the subject eye, and a scan unit that scans the illumination light formed in the slit shape on the fundus in a direction orthogonal to a slit, and the light projection and reception separating means forms the light projection regions at two positions separated from each other in a scan direction of the scan unit on the pupil of the subject eye, and forms the light receiving region to be interposed between the two light projection regions on the pupil of the subject eye.

The imaging control means may capture a first fundus image which is a fundus image based on the illumination light projected from one of the two light projection regions, and a second fundus image that is a fundus image based on the illumination light projected from the other of the two light projection regions, after alignment to the first reference position is completed, and the fundus imaging device further includes image processing means that generates a composite image with using at least two of the first fundus image and the second fundus image.

### Brief Description of Drawings

[FIG. 1] FIG. 1 is a diagram illustrating forms of irradiation and scanning of illumination light on a fundus, which is made by an imaging optical system.
FIG. 2 is a diagram illustrating a light projection region and a light receiving region formed on a pupil of a subject eye by the imaging optical system.
FIG. 3 is a diagram illustrating a fundus image captured by simultaneously irradiating the fundus with illumination light from two light projection regions.
FIG. 4 is a diagram illustrating a fundus image captured by selectively irradiating the fundus with illumination light from one of the two light projection regions.
FIG. 5 is a diagram illustrating a device configuration capable of changing clearance between the light projection region and a light receiving region.
FIG. 6 is a diagram illustrating an outline of processing when forming a composite front image in a first composition method.
FIG. 7 is a diagram illustrating an outline of processing when forming a composite front image in a second composition method.
FIG. 8 is a diagram illustrating a method of imaging a small pupil eye by changing a positional relationship between the subject eye and an imaging unit (imaging optical system).
FIG. 9 is a diagram illustrating an exterior configuration of a device according to an example.
FIG. 10 is a diagram illustrating an optical system housed in an imaging unit of an example.
FIG. 11 is a diagram illustrating an optical chopper capable of being applied as a scan unit.
FIG. 12 is a block diagram illustrating a control system of a device according to an example.
FIG. 13 is a flowchart illustrating an imaging operation of the device.
FIG. 14 is a flowchart subsequent to FIG. 13.
FIG. 15 is a diagram illustrating a display screen of a fundus observation image.

### Description of Embodiments

Hereinafter, embodiments of a fundus imaging device according to the present disclosure will be described with reference to the drawings. Hereinafter, a device, which suppresses an artifact generated by reflection and scattering in a subject eye or inside a device, is disclosed as a first embodiment. Further, a device, which can excellently capture a fundus image even in a case where a pupil diameter of a subject eye is small, is disclosed as a second embodiment. Each embodiment may appropriately use a part of other embodiments.

### <First Embodiment>

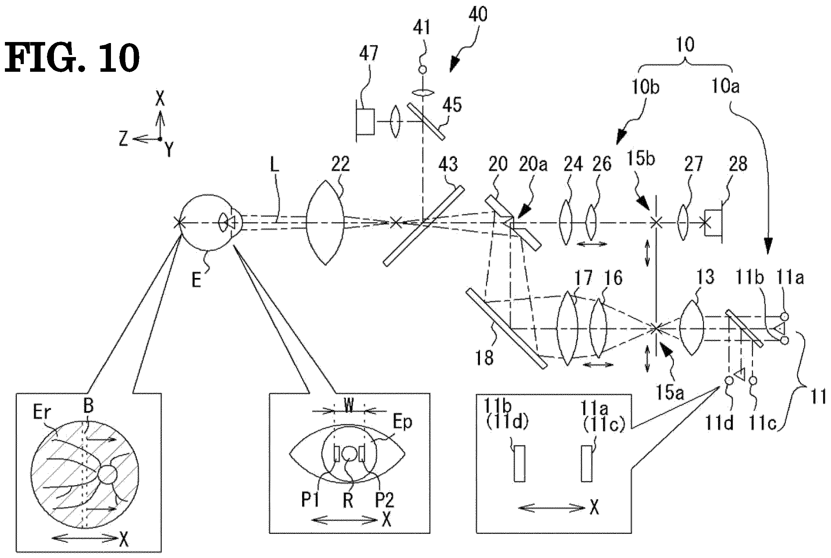

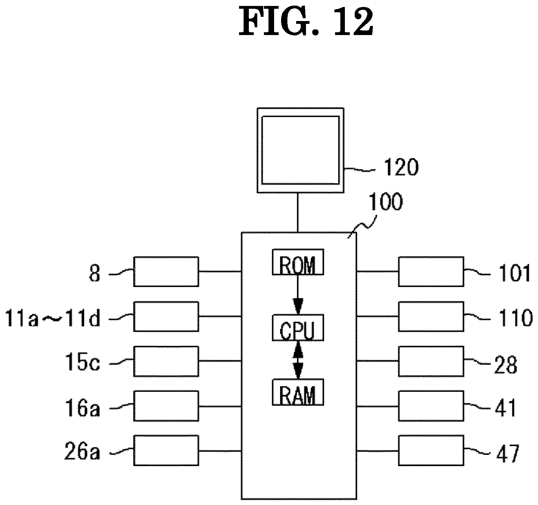

First, a first embodiment will be described. In the first embodiment, a fundus imaging device includes at least an imaging optical system (see FIG. 10) and a control unit (see FIG. 12). Additionally, the fundus imaging device may include an image processing unit.

### <Imaging Optical System>

The imaging optical system is used to capture a fundus image by projecting and receiving illumination light to and from a fundus of a subject eye. More specifically, the illumination light is projected such that the illumination light is formed in a slit shape on the fundus of the subject eye. The slit-shaped illumination light is scanned on the fundus. A fundus image is captured in an imaging area set as a scan area.

The imaging optical system includes at least a slit formation unit, a scan unit, and a light projection and reception separating unit. Additionally, the imaging optical system may include a light source, an imaging element, an optical path branching unit, and the like.

The slit formation unit forms illumination light in a slit shape on a fundus of a subject eye. For example, the slit formation unit may be configured such that a slit-shaped light transmission portion (for example, an opening) is disposed in a surface that conjugates with the fundus.

In the present disclosure, the "conjugate" is not be limited to a complete conjugate relationship and is assumed to include "approximately conjugate". That is, a case where a position is displaced from a complete conjugate position in a range permitted in relation to a technical significance of each unit is also included in the "conjugate" in the present disclosure.

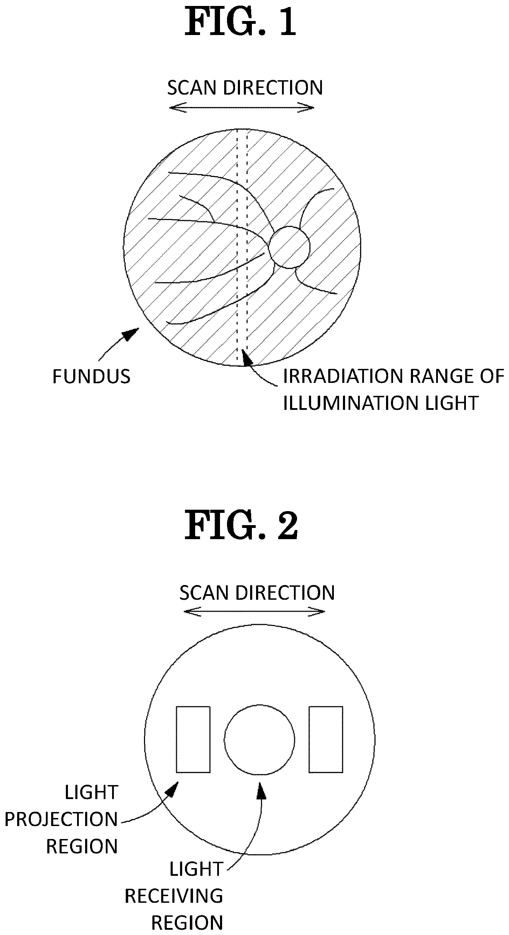

As illustrated in FIG. 1, the scan unit scans illumination light formed in the slit shape on the fundus in a direction (specifically, a direction that intersects a longitudinal direction of the slit) that intersects a slit. The scan unit may move the slit formation unit in the direction that intersects the slit to scan the illumination light. As the scan unit, a mechanical shutter, a liquid crystal shutter, an optical chopper, a drum reel, or the like may be used.

A scan direction of the slit is preferably a direction orthogonal to the slit. However, the scan direction may be a direction inclined to the orthogonal direction of the slit.

Further, the scan unit may be a member that changes a direction of light passing through the slit formation unit. For example, various optical scanners such as a galvanometer scanner may be used as the scan unit. The scan unit of a type turning light to perform scan, as exemplified as a galvanometer scanner may be placed at a position conjugating with a pupil of a subject eye.

The imaging optical system may further include an optical path coupler and an objective lens.

The optical path coupler couples and decouples a light projection optical path of illumination light and a light receiving optical path of fundus reflection light. The objective lens is disposed on a common optical path of the light projection optical path and the light receiving optical path, which is formed by the optical path coupler. At this time, it is desirable that an optical axis (hereinafter, also referred to as an "imaging optical axis") of the imaging optical system and the optical axis of the objective lens match each other.

Various beam splitters can be used as the optical path coupler. In this case, the optical path coupler may be a perforated mirror, may be a simple mirror, may be a half mirror, or may be other beam splitters.

### <Separation of Light Projection and Light Reception on Eye Pupil of Subject>

A light projection and reception separating unit separates a region (light projection region) through which the illumination light is projected on a pupil of a subject eye, and a region (light receiving region) from which fundus reflection light generated from the illumination light is extracted on the pupil of the subject eye.

Specifically, as illustrated in FIG. 2, the light projection region is formed at two positions separated from each other in a scan direction of illumination light by the light projection and reception separating unit. The two light projection regions may be formed to interpose the imaging optical axis therebetween. In the first embodiment, the light projection and reception separating unit may have at least two light projection regions formed therein and may have three or more light projection regions formed therein. Illumination light passing through each of the light projection regions is applied to the same slit-shaped region on the fundus. Then, along with driving of the scan unit, the slit-shaped region is scanned.

As illustrated in FIG. 2, the light receiving region is formed to be interposed between the two light projection regions by the light projection and reception separating unit. That is, the respective regions are formed in one line in the order of one light projection region, a light receiving region, and the other light projection region. Further, the light receiving region may be formed on the imaging optical axis. The light projection regions and the light receiving region may be disposed so as not to overlap each other. In this case, some of the illumination light is reflected and scattered and occurrence of flare in the fundus image is reduced on a cornea and an intermediate light transmission body.

The light projection and reception separating unit may include a plurality of members that are respectively disposed on the light projection optical path of the illumination light and the light receiving optical path thereof.

Some of the light projection and reception separating unit may set an irradiation position of illumination light at at least two positions separated from each other in a scan direction of the illumination light, for example, on a pupil conjugate surface of the light projection optical path of the illumination light. In this case, a light source that emits illumination light may be disposed at each of the two irradiation positions, or an opening that allows the illumination light to pass therethrough may be disposed at each of the two irradiation positions.

In other words, the light projection and reception separating unit may include at least two illumination light sources or two apparent illumination light sources, which are disposed at different positions in a scan direction at a position conjugating with a pupil of a subject' eye. Thereby, the light projection regions are formed at two positions separated from each other in the scan direction of the illumination light. More preferably, two illumination light sources or two apparent illumination light sources may be symmetrically disposed with respect to an imaging optical axis. Thereby, the two light projection regions can be symmetrically formed with respect to the imaging optical axis. A light projection state from the two light sources or two apparent light sources may be controlled for each light source by a control unit which will be described below. As a result of the control of the light projection state for each light source, whether or not to make the illumination light pass therethrough is individually set for each light projection region. The light projection and reception separating unit may include three or more illumination light sources or three or more apparent illumination light sources.

The light projection state may include at least two kinds of states, which are a state in which illumination light from the light sources or the apparent light sources reaches a subject eye and a state in which the illumination light does not reach the subject eye. Switching of the light projection state may be performed by a lighting control of the light source. Further, the switching of the light projection state may be performed by driving and controlling a shutter that selectively blocks a light flux traveling from the light source or the apparent light source toward the subject eye.

Further, the light projection and reception separating unit may be configured such that a part thereof make fundus reflection light from a light receiving region that is a region interposed between two light projection regions pass therethrough toward an imaging surface and does not make the other light pass therethrough toward the imaging surface, on a pupil conjugate surface of a light receiving optical path of the illumination light. For example, the light projection and reception separating unit may include a light blocking member that makes the fundus reflection light from the light receiving region pass therethrough toward the imaging surface and blocks the other light. The light blocking member may be disposed on, for example, a pupil conjugate surface on the light receiving optical path. For example, in a case where an aperture having an opening around an imaging optical axis is provided as the light blocking member, the light receiving region is formed by an opening image of the aperture.

In a case where the light blocking member is included in the light projection and reception separating unit, the light blocking member may be used together with the above-described optical path coupler or may be used as a separate member from the optical path coupler.

### <Control Unit>

The control unit is a processing device (processor) that performs control processing of each unit and arithmetic processing. For example, the control unit is configured by a central processing unit (CPU), a memory, and the like.

In the first embodiment, the control unit sets whether or not to pass illumination light, individually for two light projection regions on the pupil of subject eye. Further, the control unit captures a fundus image based on illumination light selectively projected from one (any) of the two light projection regions.

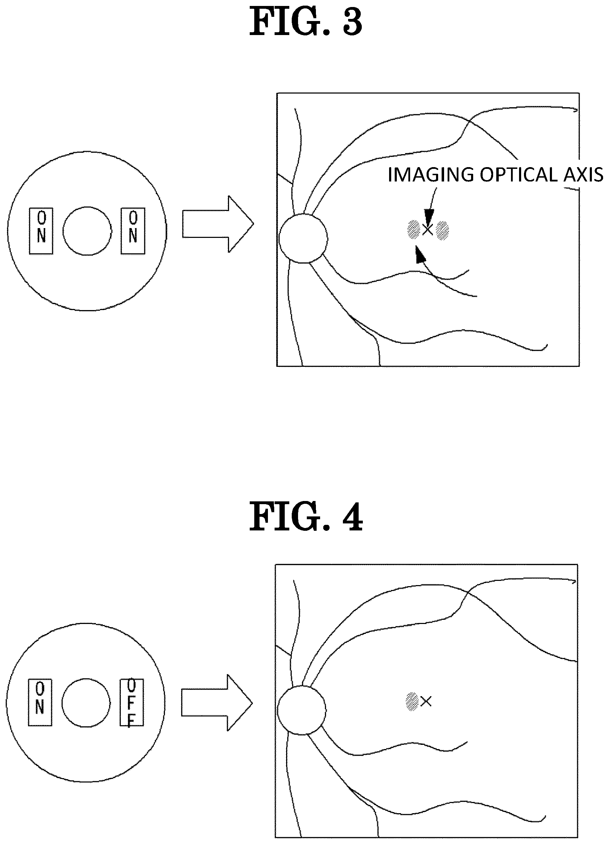

Then, there is a case where a reflection image (bright spot image, a kind of artifact) is generated due to reflection of illumination light from a central portion of a lens surface of an objective lens at a position near an imaging optical axis in the fundus image. Since the light projection region is spaced from the imaging optical axis, the reflection image easily appears at a position that slightly deviates from a position of the imaging optical axis in the fundus image. As an example, a fundus image captured by simultaneously projecting illumination light from both the two light projection regions is illustrated in FIG. 3. As illustrated in FIG. 3, a reflection image can be generated at two positions interposing the imaging optical axis therebetween in an image central portion of the fundus image, corresponding to the two light projection regions. The two reflection images appear side by side (that is, at different positions in a scan direction) in the scan direction.

In contrast to this, in the present embodiment, the illumination light is selectively projected from one of the two light projection regions to the fundus, and thus, a fundus image is captured. Thereby, a place where the reflection image is generated is halved in the objective lens as compared with the above-described case, and thus, the reflection image in the fundus image can be halved as illustrated in FIG. 4. As such, capturing a fundus image by projecting selectively the illumination light to the fundus from one of two light projection regions on the pupil of the subject eye is advantageous in reducing an artifact.

Capturing the fundus image by projecting selectively the illumination light to the fundus from one of the two light projection regions is advantageous in a case where the subject eye with a small pupil diameter is imaged. Even in a case where the pupil diameter is small and two light projection regions cannot be satisfactorily disposed inside a pupil region, if the pupil has a diameter that allows disposition of one light projection region and one light receiving region, the fundus image can be captured satisfactorily.

### <Change in Clearance between Light projection region and light receiving Region>

The light projection and reception separating unit may be changeable a clearance between the two light projection regions and the light receiving region on the pupil of the subject eye. The larger the clearance, the less likely the reflection image from the objective lens occurs. If the clearance is small, the light projection region and the light receiving region are closer to each other, which is advantageous for imaging a small pupil eye.

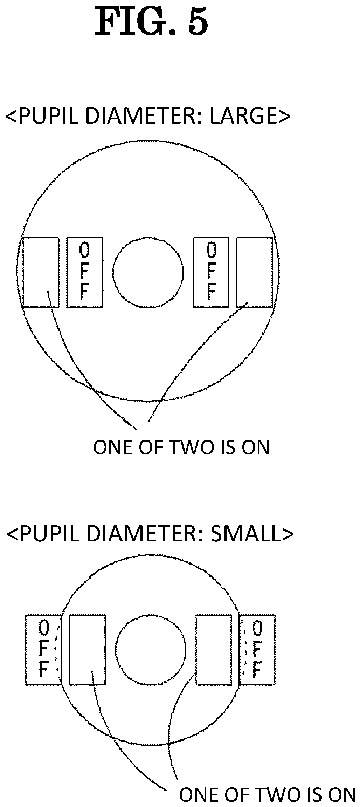

In this case, for example, in a case where a light source is disposed at a position on a pupil conjugate surface separated from an imaging optical axis to form a light projection region on a pupil of a subject eye, two sets of light sources, each set including two light sources, may be disposed as illustrated in FIG. 5. Distances from the imaging optical axis to the light sources may be different between the two sets. Further, all the light sources are disposed side by side in a scan direction. FIG. 5 illustrates images of two sets of light sources formed on the pupil of the subject eye and an opening image of an aperture. In this case, the light projection region is formed on the pupil by images of the two sets of light sources, and the light receiving region is formed by the opening image of the aperture.

Which one of the two sets is used for imaging may be selected according to the pupil diameter of the subject eye. That is, in a case where the pupil diameter is sufficiently large, the light source may be selected from one set disposed outside. In this case, a reflection image is easily reduced. Further, in a case where the pupil diameter is small, the light source may be selected from one set disposed more inside.

Further, for example, the clearance may be changed by switching a size or a shape of the light receiving region. For example, in a case where the light receiving region is formed as the opening image of the aperture, the clearance may be changed by switching one aperture disposed on an optical path among a plurality of apertures having different sizes of openings. Further, the clearance may be changed by using a variable opening aperture as the aperture.

### <Removing Artifact through Image Processing>

The control unit may capture the second fundus image by selectively projecting the illumination light from one of the two projection regions to the fundus to capture a first fundus image, and then, by further switching the light projection region to which the illumination light is projected to the other, and selectively projecting the illumination light from the other. With the two times of imaging, two fundus images can be obtained in which appearance positions of the artifacts of a reflection image and the like due to the objective lens differ from each other depending on the light projection region used for imaging. Between the first imaging and the second imaging, the imaging may be performed without changing a positional relationship between the subject eye and the imaging optical system. Further, it is preferable that the imaging of the second fundus image is consecutively and automatically performed subsequent to the imaging of the first fundus image.

In the present embodiment, when capturing two fundus images, there is no need to change a positional relationship between a subject eye and an imaging optical system, and moreover, it is possible to switch the light projection region on the pupil of the subject eye in a relatively short time by switching lighting of a light source or driving a shutter. Thus, two fundus images can be captured in a short time. As a result, even if the first fundus image and the second fundus image are consecutively captured, a burden of an examinee is suppressed. Further, even if illumination light is visible light, the second imaging is rapidly performed after the first imaging, and thus, it is possible to reduce an influence of miosis caused by the first imaging on the second imaging.

Here, an example and the like are illustrated in which vignetting of illumination light and fundus reflection light in an iris causes a fundus image to be darkened as an influence of miosis. The influence (change in brightness) of the vignetting between an image central portion and an image peripheral portion are not necessarily uniform. For example, in the image peripheral portion, as an imaging field angle becomes larger, the influence of the vignetting can be easily larger.

In the present embodiment, one fundus image (hereinafter, referred to as a "composite image") may be generated by performing synthesis processing of the two fundus images. The synthesis processing is performed by an image processing unit. The image processing unit may be an image processing processor separate from the control unit, or a part or all of the image processing unit may be shared by the processor of the control unit.

In the present embodiment, the synthesis processing is performed to obtain an image in which an influence of artifacts is suppressed. A composition method includes various methods as exemplified below.

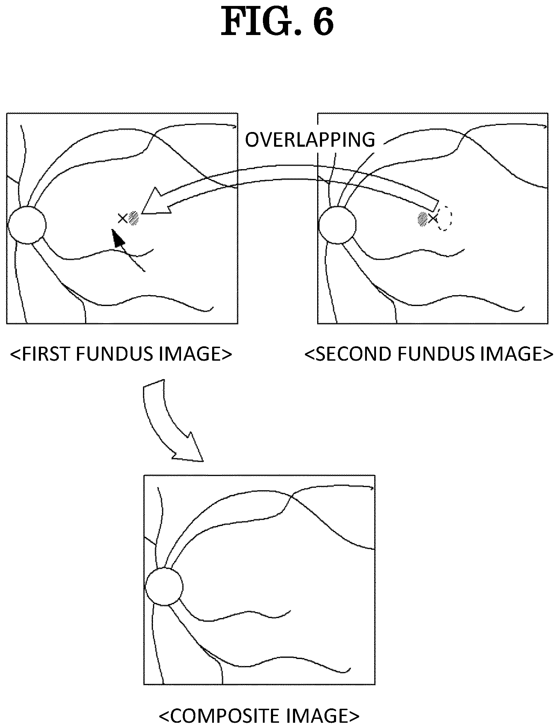

For example, the composite image may be generated by replacing a region including an artifact in one of the two fundus images with a part of the other fundus image corresponding to the region (see FIG. 6). At this time, replacement may be performed in a scan line unit. For example, in a case where the above-described reflection image is generated as an artifact, a composite image may be generated by replacing a reflection image generated at an image central portion of one fundus image with the corresponding region in the other fundus image.

Further, the composite image may be generated as an addition average image of two fundus images. In this case, addition average processing may be performed by assigning different addition rates between the reflection image and a region other than the reflection image.

The region where the reflection image is generated has some fluctuation depending on a diopter but has an approximately constant area around the imaging optical axis. Accordingly, in two fundus images, a region to be synthesized with the other image in the synthesis processing may be determined previously. However, the embodiment is not limited thereto, detection processing of the reflection image may be performed for the fundus images, and the region to be synthesized may be individually set based on a result of the detection processing. Further, the region to be synthesized may be set according to the diopter. Further, performing/non-performing of the synthesis processing may be selected according to the diopter of a subject eye.

The control unit may capture the first fundus image based on scan of only a part of a scan area corresponding to the composite image, may switch the light projection region, may capture the second fundus image based on scan of the remaining part of the scan area, and may synthesize (collage) the respective fundus images, thereby, generating the composite image. In this case, the scan area is divided into two sections about the imaging optical axis, and it is preferable that the fundus images are respectively captured in the two divided scan areas. In this case, the synthesis processing is not limited to the image processing. For example, a composite image may be formed on an imaging surface by switching the light projection region to which illumination light is projected at a timing when the illumination light arrives at a divided position of the scan area.

In this case, between two fundus images, it is preferable that a relationship between the scan area on the fundus and the light projection region to which illumination light is projected intersects between two fundus images. For example, in a case where two light projection regions are disposed side by side in a vertical direction and illumination light is scanned in the vertical direction on the fundus, when the illumination light passes through from the light projection region on an upper side, the first fundus image is captured based on scan of a lower half of the fundus, and when the illumination light passes through from the light projection region on a lower side, the second fundus image is captured based on scan of an upper half of the fundus, and thereby, a composite image may be generated from both the fundus images. In this case, in a relationship between the respective light projection regions, the reflection image of the objective lens and an image obtained by imaging a portion wherein an artifact such as flare is not easily included are synthesized. Accordingly, it is possible to obtain a composite image in which the artifact is suppressed.

Instead of the synthesis processing, the following imaging control may be performed. That is, the light projection region may be switched by the control unit at a timing when the illumination light that scans the fundus during imaging arrives at boundary positions divided by two. A composite image in which a reflection image is suppressed is generated without necessity of the synthesis processing.

### <Synthesis of Three Fundus Images>

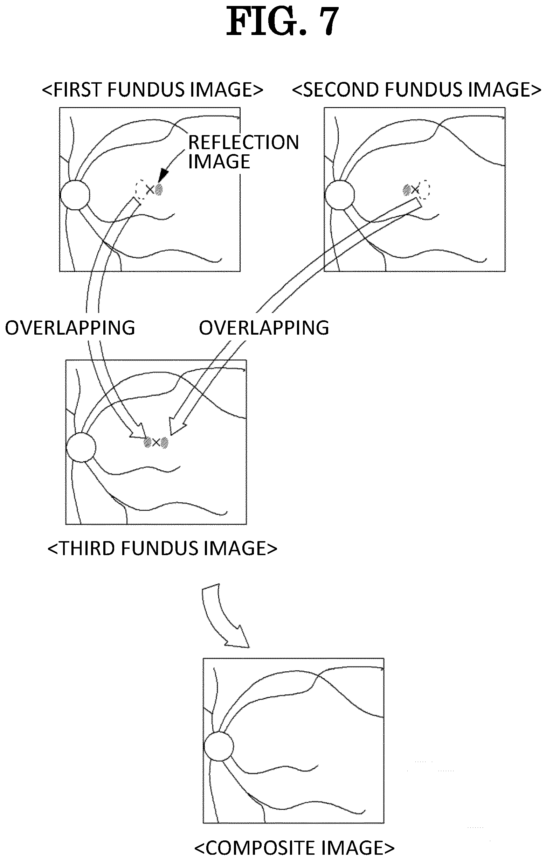

As illustrated in FIG. 7, in addition to the first fundus image captured by selectively emitting illumination light from one of the two light projection regions and a second fundus image captured by selectively emitting illumination light from the other, a third fundus image captured by simultaneously emitting the illumination light from both of the two light projection region is further captured, and a composite image in which an artifact is suppressed may be generated by synthesizing the three fundus images. In this case, a composite front image may be generated by replacing an image central portion (regions of two reflection images) in the third fundus image with a part of a corresponding region in the first fundus image and a part of a corresponding region in the second fundus image.

Since the third fundus image is captured by simultaneously emitting the illumination light from both of the two light projection regions, unevenness of brightness is reduced compared with the first and second fundus images. Since the composite image is obtained by changing only a peripheral region of the artifact in the third fundus image from the third fundus image, it is possible to suppress the unevenness of brightness.

### <Correction Processing>

In the above-described synthesis processing, various types of correction processing may be performed when synthesizing a plurality of fundus images. For example, shading may be performed to smooth a seam. Further, a difference in brightness between images may be corrected. The brightness correction is useful in reducing an influence of miosis when capturing a plurality of fundus images with using visible light.

### <Alignment between Images>

An alignment (image registration) of each fundus image may be performed by the image processing unit during the synthesis processing. The alignment referred to here may include at least one of parallel movement, rotation, expansion and contraction, affine transformation, non-linear deformation, and the like. For example, any one of phase information in a fundus image, position information of a characteristic portion (for example, any of a blood vessel, a macula, a papilla, and so on), and so on may be used for the alignment.

### <Regarding Imaging Order>

As in the above-described synthesis processing for replacement, in a case where one of a plurality of fundus images used for the synthesis processing is set as a base image and the other fundus images are synthesized with only a region near an artifact in the base image to generate a composite image, which of the plurality of fundus images is set as the base image and which are uses as the other fundus image may be selected according to an imaging order of the respective images.

For example, in a case where a plurality of fundus images are consecutively captured with using visible light as illumination light, miosis starts immediately after the first image is captured, and thus, there is a possibility that the fundus image captured thereafter are influenced by the miosis. Therefore, in the synthesis processing described above, a composite image in which an influence of the miosis is reduced is obtained by using the first image captured first as a base image.

### <Application to Other Artifacts>

In the above description, a case where a reflection image generated by a lens surface of an objective lens is suppressed by synthesis processing is described, but the above-described synthesis processing can also be applied to artifacts other than the reflection image. Specifically, the first fundus image captured by selectively emitting illumination light from one of two projection regions and the second fundus image captured by selectively emitting the illumination light from the other, the synthesis processing can be applied to artifacts generated at different positions. As one example, the above-described synthesis processing can be applied to eyelashes, flares, and the like.

### <Second Embodiment>

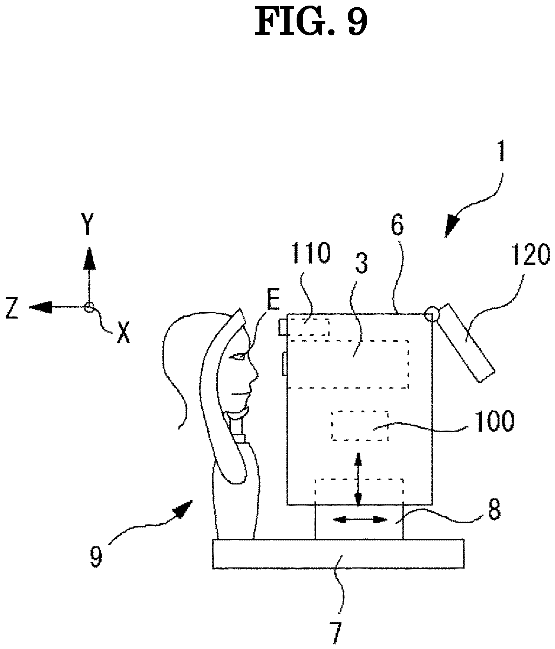

Next, a second embodiment will be described. In the second embodiment, a fundus imaging device may include at least an imaging optical system (see FIG. 10), a drive unit (see FIGS. 9 and 12), and a control unit (see FIG. 12). Additionally, the fundus imaging device may include at least one of an anterior eye segment observation optical system, an input interface, and a monitor.

### <Imaging Optical System>

The imaging optical system according to the second embodiment is used for capturing a fundus image by projecting and receiving illumination light to and from a fundus of a subject eye. The imaging optical system according to the second embodiment includes at least a light projection and reception separating unit and a light receiving element. Additionally, the imaging optical system may include at least one of a light source, an objective optical system (for example, an objective lens), an optical path branching unit, and the like.

In the second embodiment, at least the same optical system as in the first embodiment can be used as the imaging optical system. However, the embodiment is not limited to this, and various optical systems can be used as the imaging optical system according to the second embodiment. More specifically, the imaging optical system may be a scan-type optical system that scans imaging light on a tissue of a subject eye to perform imaging or may be a non-scan-type optical system. For example, the scan-type optical system may be a spot-scan-type optical system that two-dimensionally scans spot-shaped imaging light on tissue, or may be a line-scan-type optical system that scans line-shaped imaging light in one direction. In this case, any one of a point light receiving element, a line sensor, a two-dimensional light receiving element (imaging element), and the like may be appropriately employed as the light receiving element. The optical system according to the first embodiment is an example of the line-scan-type optical system. Further, for example, an optical system of a general fundus camera, or the like may be used as the non-scan-type optical system.

The light projection and reception separating unit according to the second embodiment separates at least a region (light projection region) to which the illumination light is projected and a region (light receiving region) from which fundus reflection light generated from illumination light is extracted, on a pupil of a subject eye. Then, the fundus reflection light extracted from the light receiving region is received by the light receiving element.

The light projection and reception separating unit may form each region on a pupil conjugate surface such that one of the light projection region and the light receiving region interposes the other. As one specific example, one region (the light projection region in the first embodiment) may be formed at two completely separated positions, and the other region (the light receiving region in the first embodiment) may be formed therebetween as in the first embodiment. Further, as another specific example, the light projection region and the light receiving region may be formed concentrically like a general fundus camera.

In the second embodiment, the light projection and reception separating unit is configured by one or more members disposed on one or both of a light projection optical path and a light receiving optical path. As in the first embodiment, the light projection and reception separating unit may include a plurality of members that are respectively disposed on a light projection optical path of illumination light and a light receiving optical path thereof. For example, an optical path branching unit that couples/branches the light projection optical path and the light receiving optical path may be used as the light projection and reception separating unit.

### <Anterior Eye Segment Observation Optical System>

The ophthalmic imaging device according to the second embodiment may include an anterior eye segment observation optical system (see FIG. 10) and may be capable of obtaining an anterior eye segment observation image via the anterior eye segment observation optical system. The anterior eye segment observation optical system may include at least an imaging element. The anterior eye segment observation image is used to adjust (that is, for an alignment, tracking, and the like) a positional relationship between a subject eye and an imaging optical system. The anterior eye segment observation image may be, for example, a front image of an anterior eye segment. During an alignment, the anterior eye segment observation image may be displayed on a monitor. Thereby, an examiner can grasp a real-time alignment state.

The anterior eye segment observation optical system may share a part of an imaging optical system and an optical system. The imaging optical system and the anterior eye segment observation optical system may be unitized and may be integrally changed in a positional relationship with a subject eye by a drive unit to be described below.

The anterior eye segment observation image may be captured by an imaging element separately from an imaging element of the imaging optical system. In addition to this imaging sensor, the anterior eye segment observation optical system may include various optical elements such as a light source.

Furthermore, a fundus imaging device may include an alignment index projection optical system that projects an alignment index onto a subject eye. Then, the alignment may be induced based on the alignment index formed on an observation image of an anterior eye segment or a fundus.

### <Drive Unit>

A drive unit is a mechanism that changes (adjusts) a positional relationship between a subject eye and an imaging optical system. By changing the positional relationship, a positional relationship between a light projection region and a light receiving region on a pupil of the subject eye and a pupil region is changed.

The drive unit may include an actuator that changes a positional relationship between a subject eye and an imaging optical system based on a signal from the control unit and may be a mechanical mechanism that changes the positional relationship between the subject eye and the imaging optical system according to a force given by the examiner.

For example, the drive unit may displace an imaging unit including an imaging optical system (and an anterior eye segment observation optical system), may displace a face support unit (for example, a chin rest) that supports a face of an examinee, or may combine both.

### <Control Unit>

In the second embodiment, a control unit performs alignment reference position setting processing and alignment induction processing. In the setting processing, the control unit sets an alignment reference position, which is a reference (reference for an alignment deviation) for an alignment between a subject eye and an imaging optical system, in consideration of vignetting of a light flux passing through a light projection region and a light receiving region. A positional relationship between the subject eye and the imaging optical system is adjusted (that is, an alignment) by targeting an alignment reference position.

The control unit may be capable of selectively setting at least one of a first reference position and a second reference position different from the first reference position. Here, the first reference position is a position on the assumption that all the light projection region and the light receiving region are formed inside a pupil region of the subject eye. The second reference position is a position on the assumption that a part of one of the light projection region and the light receiving region is formed outside the pupil region of the subject eye. The second reference position is a position on the assumption that the remaining part of the light projection region and the light receiving region (at least a part of each of the light projection region and the light receiving region) is formed inside the pupil region.

For example, each of the first reference position and the second reference position may be previously determined or may be settable for each subject eye. For example, each of the first reference position and the second reference position may be set based on an observation image of an anterior eye segment or a fundus. The fundus observation image is a kind of a fundus image obtained via the imaging optical system and may be a moving image captured with using invisible light such as infrared light as illumination light.

The first reference position may be a position on the assumption that the light projection region and the light receiving region are all formed inside the pupil region, for example, in the required pupil diameter (design value). In this case, for example, the first reference position may be a position in which an imaging optical axis matches the center (for example, an apex of a cornea or the center of a pupil) of a subject eye.

The second reference position may be a position that is offset in a direction in which a positional relationship between the subject eye and the imaging optical system intersects an imaging optical axis with respect to the first reference position. Further, the second reference position may be a position different from both the position in which the imaging optical axis matches the apex of the cornea, and the position in which the imaging optical axis matches the center of the pupil. Since the positional relationship between the light projection region and light receiving region and the pupil region of the subject eye changes due to the offset differently from a case of the first reference position, it is possible to improve the influence of vignetting.

The direction and amount of offset of the second reference position with respect to the first reference position may be appropriately set within a range in which a part of each of the light projection region and the light receiving region formed outside the pupil region of the subject eye, and the remaining part (at least each part of the light projection region and the light receiving region) is formed inside the pupil region, for example, based on the pupil diameter and the first reference position. At this time, the pupil diameter used in determining the offset may be, for example, a design value or an actual measurement value.

Further, at least one of the direction and amount of the offset of the second reference position with respect to the first reference position may be previously determined according to the positional relationship between the light projection region and the light receiving region. For example, the direction of the offset may be set to a direction in which a ratio between the light projection region and the light receiving region included in the pupil region changes.

Further, at least one of the direction and the amount of the offset may be set based on an observation image of an anterior eye segment or a fundus. For example, an area ratio between the light projection region and the light receiving region disposed inside the pupil region based on an anterior eye segment image is calculated, and at least one of the direction and the amount of the offset may be set according to the area ratio.

Here, an alignment reference position may be set in consideration of vignetting due to an iris. Instead of this or additionally, the vignetting due to an eyelid may be considered when setting the alignment reference position. A state of the vignetting may be grasped from an observation image of, for example, the anterior eye segment or the fundus.

Here, the control unit may set the alignment reference position based on information regarding the pupil region of the subject eye. The information regarding the pupil region may be, for example, at least information indicating a size of the pupil region (specifically, area information of the pupil region, pupil diameter information, and the like). Further, the information regarding the pupil region may be information indicating a position and a shape of the pupil region. The information regarding the pupil region can be detected based on, for example, an anterior eye segment observation image. The light projection region and the light receiving region are formed on the pupil of the subject eye at substantially known positions and sizes. Accordingly, whether or not light projection and reception are performed more appropriately can be determined based on information regarding the pupil region in either case of the first reference position and the second reference position. Then, for example, the control unit may selectively select one of the first reference position and the second reference position in which both the light projection region and the light receiving region can be evenly disposed inside the pupil region.

The information regarding the pupil region may be acquired from the anterior eye segment observation image or may be acquired as a result of measurement or imaging made by another device. Various types of processing are known as a method of setting the pupil region from the anterior eye segment observation image and can be appropriately used. Further, in a case of being acquired based on the measurement or imaging made by another device, the information regarding the pupil region may be acquired through any one of a network coupled to the ophthalmic imaging device, an external storage medium, an input interface, and the like.

Further, the control unit may set the alignment reference position based on the information regarding brightness of the fundus observation image. In the present embodiment, at least the second reference position may be set based on the information regarding the brightness of the fundus observation image. The information regarding the brightness may be, for example, information based on various statistics acquired from a histogram of luminance values of the fundus image. Here, the less the effect of vignetting, the brighter the fundus observation image becomes, and unevenness in brightness at each position is reduced. Then, for example, the control unit may acquire a plurality of fundus observation images in a plurality of alignment states in which positional relationships between eyes to be examined and imaging optical systems are different from each other, and may set the alignment reference position based on information regarding brightness of the plurality of fundus observation images. For example, the control unit may predict a position in a fundus image where a luminance distribution is maximized from the plurality of fundus observation images and may set the position as the alignment reference position. Further, for example, the control unit may predict a position in the fundus image where an average luminance value is maximized from the plurality of fundus observation images and may set the position as the alignment reference position. In this case, a positional relationship in which efficiency of light projection and reception becomes better can be set as the alignment reference position.

The control unit induces an alignment based on an alignment deviation from the alignment reference position. Here, the induction of an alignment may be performed by using a so-called automatic alignment method or a manual alignment method. In the automatic alignment method, the control unit may perform a drive control of a drive unit based on the alignment deviation from the alignment reference position.

Further, in the manual alignment method, the control unit may display an anterior eye segment observation image on a monitor and may display a guide (for example, an electronic reticle) for guiding an operation to a target position on the anterior eye segment observation image, based on the set alignment reference position. In this case, the ophthalmic imaging device may include an operation input unit that receives an operation of an examiner and drives the drive unit according to the operation to adjust a relative position. The operation input unit may be an input interface to which an operation for driving an actuator of the drive unit is input or may be a unit that directly acts on a mechanical drive unit.

As a result of the alignment induction, the alignment may be completed in a step in which the deviation from the alignment reference position falls within an allowable range. Subsequently, fine adjustment, diopter correction, and the like based on the fundus observation image may be performed. Then, after completion of various adjustments, the control unit may automatically perform fundus imaging. Further, in a case where imaging is performed based on a release operation by an examiner, a display prompting the release operation may be displayed on a monitor.

### <Alignment Reference Position Setting According to Presentation Position of Fixation Target>

Further, setting of the alignment reference position may be performed in conjunction with a presentation position of a fixation target. In this case, an ophthalmic imaging device may further include a fixation optical system that presents the fixation target to a subject eye. The fixation optical system may be changeable an orientation of fixation by switching a position of the fixation target.

By switching the presentation position of the fixation target and changing an angle between a visual axis of a subject eye and an imaging optical axis, a shape and the like of a pupil region on a pupil conjugate surface is changed. The control unit can satisfactorily capture a fundus image of the subject eye at the presentation position of the fixation target by setting the alignment reference position according to the presentation position of the fixation target.

### <Operation in Mode in Which Light Projection and Reception Separating Unit Forms at Least Two Light Projection Regions>

As in the first embodiment, the light projection and reception separating unit may form at least two light projection regions having different positions on a pupil of the subject eye. The light receiving region is preferably formed to be interposed between two light projection regions.

In this case, at the second reference position, one of the two light projection regions together with the light receiving region may be in a position disposed inside the pupil region of the subject eye more preferentially than the remaining one light projection region. The control unit may select one of at least two of the first reference position and the second reference position to induce an alignment. The first reference position may be a position in which the center of the subject eye (for example, one of an apex of a cornea and the center of a pupil, or the like) and the imaging optical axis matches each other, and the first reference position may be a position in which the center of the subject eye and a position of the center of gravity of two of the light projection region and the light receiving region match each other.

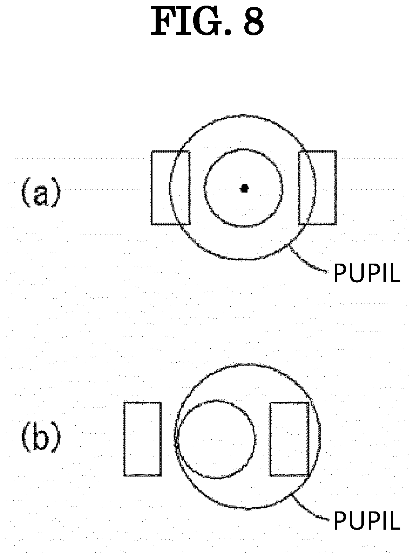

As such, in a case where an alignment is performed with the first reference position as a reference, two light projection regions are evenly disposed inside a pupil region of a subject eye together with the light receiving region, and thus, a satisfactory fundus image is easily captured with the sufficient amount of light in a case where the pupil region of the subject eye is large enough. However, it is considered that it is difficult to dispose simultaneously both of two light projection regions inside the pupil in a case where a pupil diameter is small, or the amount of illumination light reaching a fundus is insufficient if each of the two light projection regions is disposed to partially overlap an iris (see (a) in FIG. 8). Then, in this case, the alignment is preferably performed with the second reference position as a reference. At the second reference position, one of the two light projection regions is disposed inside the pupil of the subject eye more preferentially than the remaining one light projection region together with the light receiving region (see (b) in FIG. 8). At this time, the remaining one light projection region may be disposed outside the pupil. In a case where the alignment to the second reference position is completed, both of one light projection region and the light receiving region are satisfactorily disposed inside the pupil, and thus, the subject eye having a smaller pupil diameter can be satisfactorily imaged.

The first reference position and the second reference position may be selected, for example, according to a size of the pupil region of the subject eye. For example, in a case where the size of the pupil region is larger than a threshold, the first reference position may be selected, and in a case where the pupil region is smaller than the threshold, the second reference position may be set. That is, a second imaging mode is used to image an eye having a relatively small pupil diameter. At this time, the threshold may be, for example, a value corresponding to a disposition interval of the two light projection regions on the pupil of the subject eye.

Further, among the two light projection regions formed on the pupil conjugate surface, regions through which illumination light passes during fundus imaging may be different between the first reference position and the second reference position. For example, in a case where the alignment is made for the first reference position, the illumination light may be projected onto the fundus from both of the two projection regions formed on the pupil conjugate surface to capture a fundus image. At this time, the fundus image may be captured by simultaneously passing the illumination light from both of the two projection regions. Further, as in the first embodiment, making the illumination light pass between the two light projection regions may be alternately switched, and a fundus image may be captured at each switching. Furthermore, a composite image may be generated from at least two fundus images captured by alternately projecting light.

Further, in a case where the alignment is made for the second reference position, illumination light may be projected onto a fundus from only one of the two light projection regions to capture a fundus image. At this time, it is preferable that the illumination light is projected from the light projection region preferentially disposed inside the pupil region among the two light projection regions.

Further, in a case where an alignment is made for the second reference position and imaging is performed, at least one of the amount of illumination light and a gain of an imaging element is increased compared with a case where an alignment is made for the first reference position and imaging is performed, and thus, the fundus image may be captured. The amount of light or the gain may be set according to a size of the pupil region. Further, the amount of light or the gain may be set depending on a ratio between a part of the light projection region and the light receiving region disposed inside the pupil region and the total area of the light projection region and the light receiving region.

### <How to Dispose Two Light Projection Regions and light receiving Region>

The light projection and reception separating unit may form the two light projection regions and the light receiving region at positions separated in a left-right direction on a pupil conjugate surface. In this case, a width in a vertical direction is easily narrowed more than the total length in the left-right direction in the three regions. As a result, eyelids are less likely to overlap the light projection regions and the light receiving region, and vignetting due to blinking is easily suppressed.

Further, the light projection and reception separating unit may form the two light projection regions at positions separated in the vertical direction on the pupil conjugate surface of the subject eye. In this case, the second reference position may be set such that the light projection region formed on a lower side among the two light projection regions is preferentially disposed inside the pupil. Then, the control unit may perform an alignment for the second reference position, and when the fundus image is captured, the control unit may project illumination light from the preferentially disposed light projection region, and thereby, the fundus image is capture. In this case, the light projection region is less likely to overlap an eyelid during blinking, and thus, vignetting due to the blinking is easily suppressed.

### <Artifact Correction of Fundus Image Captured at Second Reference Position>

In a case where the light projection and reception separating unit forms at least two light projection regions, the control unit may further select a third reference position as the alignment reference position to capture a fundus image.

Here, the third reference position is a position in which the other light projection region is preferentially disposed together with the light receiving region, compared with one light projection region which is preferentially disposed in the pupil region at the second reference position among the two light projection regions.

The control unit may first induces an alignment in the second reference position, capture the first fundus image based on at least illumination light from the light projection region preferentially disposed in the pupil region at the second reference position among the two light projection regions, and thereafter, further induce an alignment in the third reference position, and capture a second fundus image based on at least illumination light from the light projection region preferentially disposed in the pupil region at the third reference position among the two light projection regions. Here, it is considered that the first fundus image and the second fundus image are different from each other in reflection images of objective lens and positions where artifacts such as flare are generated. Therefore, as in the first embodiment, a composite image of two fundus images may be generated.

### <Examples>

Next, examples of fundus imaging devices according to the first embodiment and the second embodiment will be described with reference to FIG. 9 to FIG. 15.

A fundus imaging device 1 (hereinafter, simply referred to as an "imaging device 1") forms illumination light on a fundus of a subject eye in a slit shape, scans a region formed on the fundus in the slit shape, receives fundus reflection light of the illumination light, thereby, capturing a front image of the fundus.

### <Exterior of Device>

An exterior configuration of the imaging device 1 will be described with reference to FIG. 9. The imaging device 1 includes an imaging unit 3. The imaging unit 3 mainly includes an optical system illustrated in FIG. 10. The imaging device 1 includes a base 7, a drive unit 8, a face support unit 9, and a face imaging camera 110, and adjusts a positional relationship between a subject eye E and the imaging unit 3 with using the above-described units.

The drive unit 8 can move in a left-right direction (X direction) and a front-rear direction (Z direction, in other words, an operation distance direction) with respect to the base 7. Further, the drive unit 8 can further move the imaging unit 3 in a three-dimensional direction with respect to the subject eye E on the drive unit 8. The drive unit 8 includes an actuator for moving the drive unit 8 or the imaging unit 3 in the respective movable directions previously determined, which is driven based on a control signal from a control unit 80. The face support unit 9 supports a face of an examinee. The face support unit 9 is fixed to the base 7.

The face imaging camera 110 is fixed to a housing 6 such that a positional relationship on the imaging unit 3 is constant. The face imaging camera 110 images the face of the examinee. A control unit 100 specifies a position of the subject eye E from a captured face image and performs a drive control of the drive unit 8, thereby, aligning the imaging unit 3 with respect to the specified position of the subject eye E.

Further, the imaging device 1 further includes a monitor 120. A fundus observation image, a fundus imaging image, an anterior eye segment observation image, and the like are displayed on the monitor 120.

### <Optical System of Example>

Next, an optical system of the imaging device 1 will be described with reference to FIG. 10. The imaging device 1 includes an imaging optical system (fundus imaging optical system) 10, and an anterior eye segment observation optical system 40. The optical systems are provided in the imaging unit 3.

In FIG. 10, a position that conjugates with a pupil of a subject eye is indicated by "Δ" on an imaging optical axis, and a fundus conjugate position is indicated by "X" on the imaging optical axis, respectively.

The imaging optical system 10 includes an irradiation optical system 10a and a light receiving optical system 10b. In the example, the irradiation optical system 10a includes a light source unit 11, a lens 13, a slit-shaped member 15a, lenses 16 and 17, a mirror 18, a perforated mirror 20, and an objective lens 22. The light receiving optical system 10b includes the objective lens 22, the perforated mirror 20, lenses 24 and 26, a slit-shaped member 15b, and an imaging element 28. The perforated mirror 20 is an optical path coupling portion that couples optical paths of the irradiation optical system 10a and the light receiving optical system 10b. The perforated mirror 20 reflects illumination light from a light source toward the subject eye E, and makes partial fundus reflection light passing through an opening among fundus reflection light from the subject eye E pass therethrough to go toward an imaging element. Various beam splitters can be used instead of the perforated mirror 20. For example, instead of the perforated mirror 20, a mirror in which a light transmission portion and a reflection portion are reversed as compared with the perforated mirror 20 may be used as the optical path coupling portion. However, in this case, an independent optical path of the light receiving optical system 10b is placed on a reflection side of a mirror, and an independent optical path of the light projection optical system 10a is placed on a light transmission side of the mirror. Further, the perforated mirror and a mirror that is alternative means thereof can be further replaced with a combination of a half mirror and a light blocking mirror, respectively.

In the present example, the light source unit 11 includes a plurality of types of light sources having different wavelength bands. For example, the light source unit 11 includes visible light sources 11a and 11b, and infrared light sources 11c and 11d. As such, the light source unit 11 according to the present example is provided with two light sources for each wavelength. Two light sources having the same wavelength are disposed to be spaced from an imaging optical axis L on a pupil conjugate surface. Two light sources are lined up in the X direction that is a scan direction of FIG. 10, and are disposed in axial symmetry with respect to the imaging optical axis L. As illustrated in FIG. 10, an outer peripheral shape of the two light sources may be a rectangular shape whose direction intersecting the scan direction is longer than the scan direction.

Light from two light sources passes through the lens 13 and is applied to the slit-shaped member 15. In the present example, the slit-shaped member 15a includes a light transmission portion (opening) that is formed narrowly and lengthways in the Y direction. Thereby, illumination light is formed in a slit shape on a fundus conjugate surface (a region illuminated in a slit shape on a fundus is indicated by a reference sign B).

In FIG. 10, the slit-shaped member 15a is displaced by the drive unit 15c such that the light transmission portion crosses the imaging optical axis L in the X direction. Thereby, the illumination light in the present example is scanned. In the present example, the scan is performed by the slit-shaped member 15b on the light receiving system side. In the present example, the slit-shaped members on the light projection side and the light receiving side are driven in conjunction with each other by one drive unit (actuator).

In the irradiation optical system 10a, an image of each light source is relayed by an optical system from the lens 13 to the objective lens 22 to be formed on a pupil conjugate surface. That is, images of two light sources are formed at positions separated in the scan direction, on the pupil conjugate surface. By doing so, in the present example, two light projection regions P1 and P2 on the pupil conjugate surface are formed as images of two light sources.

Further, slit-shaped light passing through the slit-shaped member 15a is relayed by an optical system from the lens 16 to the objective lens 22 to be formed on a fundus Er. Thereby, illumination light is formed in a slit shape on the fundus Er. The illumination light is reflected on the fundus Er and is extracted from a pupil Ep.

Here, since an opening of the perforated mirror 20 conjugates with a pupil of a subject eye, the fundus reflection light used for capturing a fundus image is limited to a part thereof that passes through an image (pupil image) of the opening of the perforated mirror on the pupil of the subject eye. As such, the image of the opening on the pupil of the subject eye becomes a light receiving region R in the present example. The light receiving region R is formed to be interposed between two light projection regions P1 and P2 (images of two light sources). Further, the light receiving region R and the two light projection regions P1 and P2 are formed so as not to overlap each other on the pupil, as a result of appropriate setting of an imaging magnification of each image, a diameter of the opening, and a disposition interval of two light sources. Thereby, occurrence of flare is satisfactorily reduced.

The fundus reflection light passing through the objective lens 22 and the opening of the perforated mirror 20 forms a slit-shaped region of the fundus Er at the fundus conjugate position through the lenses 24 and 26. At this time, the light transmission portion of the slit-shaped member 15b is disposed at a position in which an image is formed, and thus, harmful light is removed.

The imaging element 28 is disposed at a fundus conjugate position. In the present example, a relay system 27 is provided between the slit-shaped member 15b and the imaging element 28, and thereby, both the slit-shaped member 15b and the imaging element 28 are disposed at the fundus conjugate position. As a result, removal of harmful light and image formation are both satisfactorily performed. Instead of this, the relay system 27 may not be provided between the imaging element 28 and the slit-shaped member 15b, and both may be disposed to be close to each other. In the present example, a device having a two-dimensional light receiving surface is used as the imaging element 28. For example, a CMOS, a two-dimensional CCD, and the like may be used therefor. An image of the slit-shaped region of the fundus Er, which is formed by the light transmitting portion of the slit-shaped member 15b, is projected to the imaging element 28. The imaging element 28 has a sensitivity in both infrared light and visible light.

In the present example, as slit-shaped illumination light is scanned on the fundus Er, images (slit-shaped images) of scan positions on the fundus Er are sequentially projected for each scan line of the imaging element 28. As such, an entire image of a scan area is projected onto the imaging element in a time-division manner. As a result, a front image of the fundus Er is captured as the entire image of the scan area.

The scan unit of the light receiving system in the present example is a device that mechanically scans a slit but is not limited thereto. For example, the scan unit on the light receiving optical system side may be a device that electronically scans the slit. As an example, in a case where the imaging element 28 is a CMOS, the slit may be scanned by a rolling shutter function of the CMOS. In this case, by displacing a region exposed on an imaging surface in synchronization with the scan unit of a light projection system, it is possible to perform efficient imaging while removing harmful light. Further, a liquid crystal shutter or the like can also be used as a scan unit that electronically scans a slit.

The imaging optical system 10 includes a diopter correction unit. In the present is example, diopter correction units are respectively provided on an independent optical path of the irradiation optical system 10a and an independent optical path of the light receiving optical system 10b. The light projection optical system 10a can change an interval between the lenses 16 and 17, and the light receiving optical system 10b can change an interval between the lenses 24 and 26, and thereby, correction diopter is performed. The imaging device 1 includes drive units 16a and 26a (see FIG. 12) for changing intervals between the respective lenses, and the respective drive units 16a and 26a of the irradiation optical system 10a and the light receiving optical system 10b are driven in conjunction with each other.

The diopter correction unit is not limited thereto. For example, the diopter correction may be performed by moving the light sources 11a to 11d, the slit-shaped members 15a and 15b, and the imaging element 28 in an optical axis direction while maintaining a positional relationship between at least three thereof.

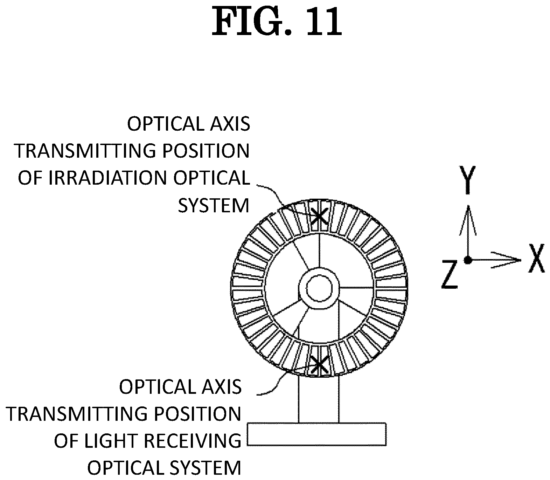

A scan unit of an ophthalmic device may be, for example, an optical chopper as illustrated in FIG. 11. The optical chopper includes a wheel in which a plurality of slits are formed on an outer periphery thereof, and the slits can be scanned at high speed by rotating the wheel.

Here, in FIG. 10, the light source unit 11 to the mirror 18 of the irradiation optical system 10a and the perforated mirror 20 to the imaging element 28 of the light receiving optical system 10b are disposed in parallel in the X direction, but for example, orientations of the perforated mirror 20 and the mirror 18 are rotated by 90° from the illustrated state and both are disposed in parallel in the Y direction, and thereby, the optical chopper can be applied as a scan unit. In this case, by causing an optical axis of the irradiation optical system 10a and an optical axis of the light receiving optical system 10b to cross in two places of an upper end and a lower end of the wheel, respectively, scans of a light projection system and a light receiving system can be easily synchronized by one optical chopper.

### <Anterior Eye Segment Observation Optical System>

Next, the anterior eye segment observation optical system 40 will be described. The anterior eye segment observation optical system 40 shares the objective lens 22 and a dichroic mirror 43 with the imaging optical system 10. The anterior eye segment observation optical system 40 further includes a light source 41, a half mirror 45, an imaging element 47, and the like. The imaging element 47 is a two-dimensional imaging element and is disposed, for example, at a position that optically conjugates with the pupil Ep. The anterior eye segment observation optical system 40 illuminates an anterior eye segment with infrared light to capture a front image of the anterior eye segment.

The anterior eye segment observation optical system 40 illustrated in FIG. 10 is only an example and may image the anterior eye segment on an optical path independent of other optical systems.

### <Control System of Example>

Next, a control system of the imaging device 1 will be described with reference to FIG. 12. In the present example, the control unit 100 controls the respective units of the imaging device 1. Further, for the sake of convenience, it is assumed that image processing of various images obtained by the imaging device 1 is also performed by the control unit 100. In other words, in the present example, the control unit 100 also serves as an image processing unit.

The control unit 100 is a processing device (processor) including an electronic circuit that performs control processing of each unit and arithmetic processing. The control unit 100 is realized by a central processing unit (CPU), a memory, and the like. The control unit 100 is electrically connected to a storage unit 101 through a bus or the like.

The storage unit 101 stores various control programs, fixed data, and the like. Further, the storage unit 101 may store temporary data or the like.

An image captured by the imaging device 1 may be stored in the storage unit 101. However, the present disclosure is not limited thereto, and the captured image may be stored in an external storage device (for example, a storage device connected to the control unit 100 through a LAN and a WAN).

Further, the control unit 100 is also electrically connected to the respective units such as the drive unit 8, the light sources 11a to 11d, the drive unit 15c, the drive unit 16a, the drive unit 26a, the imaging element 28, the light source 41, the imaging element 47, the input interface 110, the monitor 120, and the like.

Furthermore, the control unit 100 controls the respective members described above based on an operation signal output from the input interface 110 (operation input unit). The input interface 110 is an operation input unit that receives an operation of an examiner. For example, the input interface 110 may be a mouse, a keyboard, or the like.

### <Operation Description of Example>

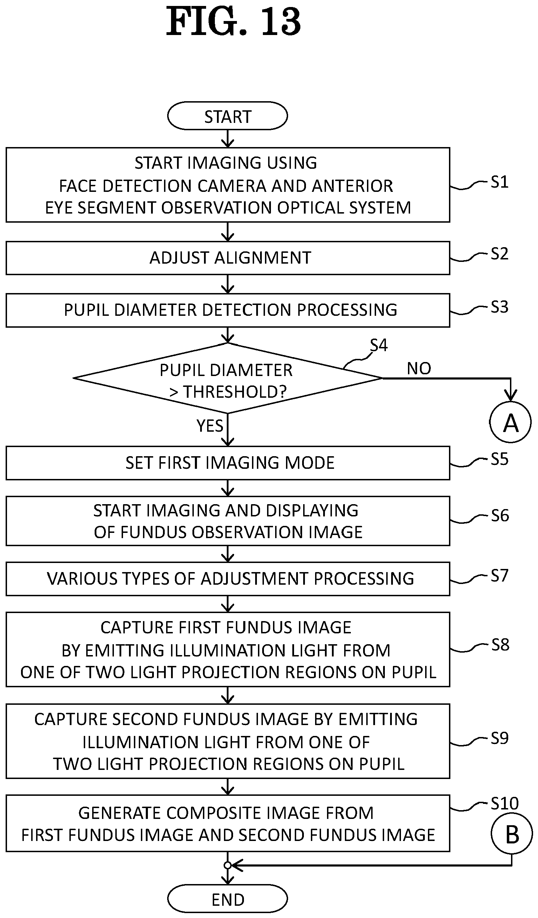

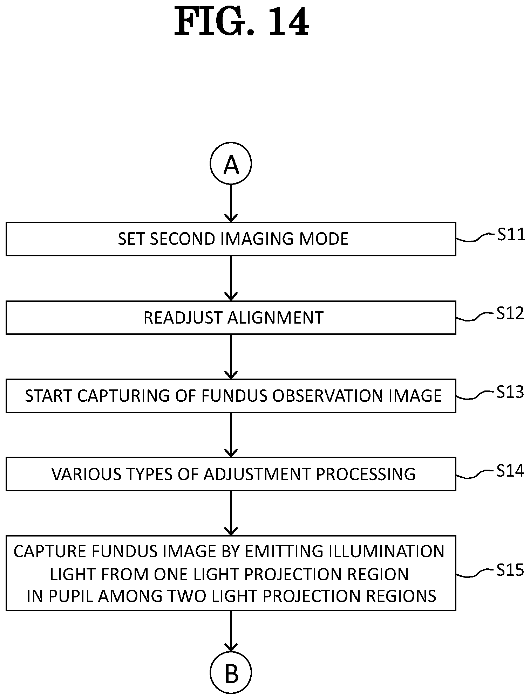

Next, an imaging operation will be described with reference to flowcharts of FIGS. 13 and 14.

The imaging device 1 may automatically start the imaging operation as a face of an examinee is disposed on a face support unit 9 and is included in an imaging area of the face detection camera 110.

First, imaging of the face detection camera 110 and imaging of the anterior eye segment observation optical system 40 are performed in parallel (S1), and an alignment adjustment is performed with using results of both imaging (S2).

Specifically, the control unit 100 detects a position of one of right and left eyes included in a face image and drives the drive unit 8 based on position information thereof. Thereby, a position of the imaging unit 4 is adjusted up to a position in which an anterior eye segment can be observed.

Next, an alignment reference position is set based on an anterior eye segment front image, and an alignment is induced in the set alignment reference position. In the present example, a positional relationship between the subject eye E and the imaging unit 3 is adjusted by the control unit 100 based on the anterior eye segment front image. In the present example, the control unit 100 may set a first reference position for targeting a positional relationship in which the center of a pupil in the front eye segment observation image and the center of an image (in the present example, a position of the imaging optical axis L) approximately match each other, based on a signal from the imaging element 47. Then, an alignment deviation from the first reference position is detected, and the imaging unit 4 is moved in vertical and left-right directions in a direction in which the alignment deviation is removed. At this time, for example, the alignment deviation with the first reference position may be detected based on the amount of deviation between the center of a pupil and the imaging optical axis on an anterior eye segment observation image. Further, in a case where the fundus imaging device 1 includes, for example, an alignment projection optical system that projects an alignment index to an apex of a cornea, the alignment deviation may be detected based on the amount of deviation between the alignment index and the imaging optical axis.

Further, the control unit 100 moves the imaging unit 4 in the front-rear direction such that the anterior eye segment observation image is focused on the pupil Ep.

As such, in the present example, as a result of the alignment adjustment of S2, a positional relationship between the subject eye and the imaging unit 4 is adjusted to a position (the first reference position in the present example) in which the center (that is, the imaging optical axis) of the light receiving region R on the pupil of the subject eye matches the center of the pupil.

In the present example, after the alignment to the first reference position is completed, pupil diameter detection processing (pupil information acquisition processing) is performed (S3). In the present example, the pupil region Ep is detected from the anterior eye segment observation image, and a diameter of the detected pupil region Ep is obtained. In the present example, a diameter in the scan direction is preferably obtained. In the present example, a value of the diameter of the pupil region Ep acquired in the processing of S3 is acquired as pupil diameter information and stored in a memory.

Next, the control unit 100 sets an imaging mode according to the acquired pupil diameter (S4, S5, and S11). In the present example, the pupil diameter is first compared with a threshold (S4). The threshold is a value corresponding to a disposition interval of the two light projection regions P1 and P2 on the pupil of the subject eye, and in the present example, a total length W (see FIG. 10) is used as an example of the threshold. Instead of the total length W, for example, a distance between the centers of the light projection regions P1 and P2 may be used as the threshold. Here, in the present example, the threshold assumed to be a fixed value regardless of a diopter correction amount. For example, a fixed value assuming zero D eye is used as the threshold.

However, since the disposition interval between the two light projection regions P1 and P2 changes depending on a diopter correction state, the threshold may be obtained each time according to a diopter error of the subject eye (or according to diopter correction amount when a slit image is formed on the fundus Er), and the obtained threshold may be compared with the pupil diameter.

As a result of the comparison between the pupil diameter and the threshold, when the pupil diameter is larger than the threshold (S4; Yes), the first imaging mode is set (S5). Meanwhile, in a case where the pupil diameter is smaller than or equal to the threshold (S4; No), a second imaging mode is set (S11).

### <First Imaging Mode>

If the pupil diameter is larger than the above-described threshold, three regions of the light projection regions P1 and P2 and the light receiving region R can be disposed inside the pupil Ep. Then, the control unit 100 drives the drive unit 8 to adjust the positional relationship between the subject eye E and the imaging unit 4 by targeting (that is, targeting the first reference position) an alignment state in which the center (in the present example, the position of the imaging optical axis L and the center of gravity of the two light projection regions P1 and P2 and the light receiving region R) of the light receiving region R substantially matches the center of the pupil.

After the first imaging mode is set, the control unit 100 starts imaging and displaying of a fundus observation image (S6). Specifically, the control unit 100 simultaneously turns on the light sources 11c and 11d, starts driving of the drive unit 15c, and repeatedly scans slit-shaped illumination light in a predetermined area on the fundus Er. For each of a predetermined number of scans (at least once), a fundus image captured in approximately real time is generated from time to time as a fundus observation image based on a signal output from the imaging element 28. The control unit 100 may display a fundus observation image on the monitor 120 as an approximately real-time moving image.

Next, various types of adjustment processing are performed based on the fundus observation image (S7). For example, diopter correction, fine adjustment of an alignment target position, and the like may be performed.

Thereafter, imaging control of the fundus imaging image is performed automatically or based on a release operation.

In the present example, the control unit 100 alternately turns on the light sources 11a and 11b that emit visible light and scans illumination light on the fundus Er every time each of the light sources 11a and 11b is turned on, and the first fundus image and the second fundus image are captured (S8 and S9).

Then, the control unit 100 synthesizes the first fundus image and the second fundus image to generate a composite image. Since a miosis starts immediately after the first fundus image is captured, there is a possibility that the second fundus image captured later among the two fundus images becomes darker than the first fundus image due to an influence of vignetting caused by the miosis.

Then, in the present example, an image, which is obtained by replacing a region of the first fundus image where a reflection image of a central portion is generated with a corresponding region in the second fundus image, is generated as a composite image. As a result, a fundus image in which the influence of the reflection image on the first fundus image and the second fundus image is suppressed can be obtained as the composite image, and thus, an influence of vignetting due to miosis can be suppressed.

### <Second Imaging Mode>

Next, an operation in a case where the second imaging mode is set will be described. If a pupil diameter is smaller than or equal to a threshold as determined in the processing of S4, three regions of the two light projection regions P1 and P2 and the light receiving region R cannot be satisfactorily disposed inside the pupil Ep. Then, the control unit 100 readjusts an alignment state. At that time, the control unit 100 sets a second reference position based on an anterior eye segment observation image. The second reference position is an alignment reference position in which one (here, the region P1) of the two light projection regions P1 and P2 is prioritized over the other and is disposed inside the pupil Ep together with the light receiving region R (S12). As an example, the second reference position is set by targeting an alignment state in which a midpoint between the light projection region P1 and the light receiving region R matches the center of the pupil. Here, for example, a positional relationship between a subject eye and an imaging optical system may be offset in the X direction with respect to the alignment reference position (that is, the first reference position) in the alignment processing of S2. Here, it is assumed that the offset is made by W/4 (see FIG. 10 for W). However, the amount of offset is not limited thereto.

Then, the control unit 100 detects an alignment deviation from the second reference position and moves the imaging unit 4 in vertical and left-right directions and in a direction in which the alignment deviation is removed. At this time, for example, an alignment deviation with the second reference position may be detected based on the amount of deviation between the center of the pupil (or the apex of the cornea) and the imaging optical axis on the anterior eye segment observation image, and the above-described offset. As a result of the alignment for the second reference position, the light projection region P1 and the light receiving region R are satisfactorily disposed inside the pupil Ep. Which of the two light projection regions P1 and P2 is preferentially disposed inside the pupil Ep may be previously determined or selectable.

After the alignment state is readjusted, a fundus observation image starts to be captured. Here, when obtaining the fundus observation image in the first imaging mode, the two light sources 11c and 11d are turned on simultaneously, but in the second imaging mode, only the light source (the light source 11c in the present example) corresponding to the light projection region P1 preferentially disposed inside the pupil Ep may be turned on. Thereby, an observation image of the fundus Er may be obtained by irradiating a fundus with illumination light for observation only from the light projection region P1. However, the present disclosure is not limited thereto, and the observation image of the fundus Er may be obtained by turning on the light sources corresponding to both the light projection regions P1 and P2.

Then, various types of adjustment processing are performed based on the fundus observation image (S14), and after the adjustment is completed, an imaging operation of the fundus imaging image in the second imaging mode is performed automatically or based on a release operation (S15).

In the second imaging mode of the present example, the control unit 100 captures a fundus image by turning on only the light source 11a, which corresponds to the light projection region P1 preferentially disposed inside the pupil Ep, among the two light sources 11a and 11b that emit visible light. The fundus image is stored in a memory as a captured image. Compared with a case where the two light sources 11a and 11b are turned on simultaneously, places where strong reflection occurs on the objective lens 22 are reduced, and thus, an influence of a reflection image on the captured image is reduced. Further, illumination light is applied to the outside of the pupil Ep such as an iris and strong film, and it is suppressed that the reflected light becomes harmful light and influences the fundus image. Thus, a good fundus image can be captured even in a case where the pupil diameter of the subject eye is small.

Here, in the present example, the control unit 100 increases at least one of the amount of light from the light sources 11a and 11b emitted at the time of imaging in the second imaging mode and a gain of the imaging element 28 that receives fundus reflection light at the time of imaging in the first imaging mode. Thereby, brightness and contrast of the fundus image captured in the second imaging mode can be increased. The amount of light and the gain may be set depending on a size (for example, a pupil diameter) of a pupil region of an examinee.

As described above, description is made based on the embodiments, but in implementing the present disclosure, the content of the embodiments can be appropriately changed.

For example, in the above examples, a positional relationship between a subject eye and an imaging unit is automatically adjusted by a control unit. However, the present disclosure is not limited thereto, and an examiner may manually adjust the positional relationship between the subject eye and the imaging unit.

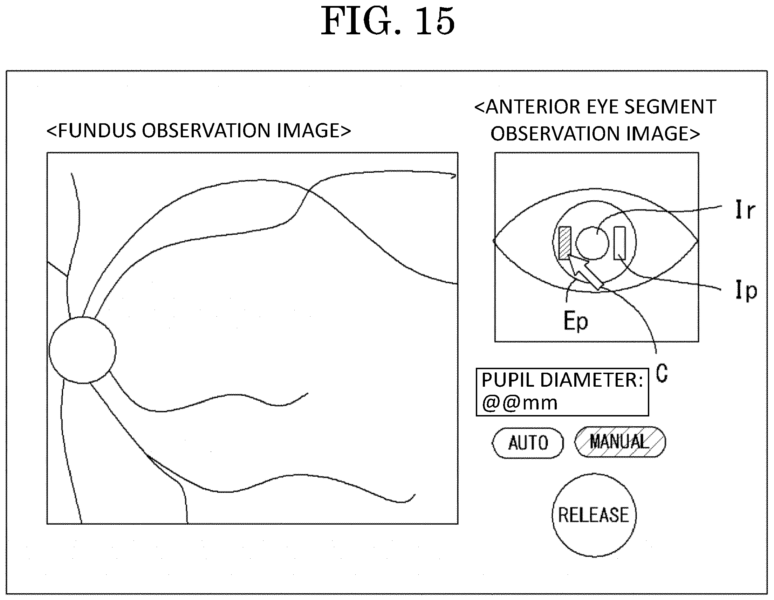

In this case, as illustrated in FIG. 15, the control unit may display at least an anterior eye segment observation image on a monitor. Thereby, the examiner can adjust the positional relationship between the subject eye and the imaging unit while viewing the anterior eye segment observation image. Further, it is preferable that an electronic index Ip corresponding to at least two light projection regions is displayed on the anterior eye segment observation image. Further, the index Ip corresponding to the light receiving region may be displayed. Positions and sizes of the indexes Ip and Ir may be constant on the anterior eye segment observation image or may be changed depending on a diopter correction amount of an imaging optical system.

By displaying the indexes Ip and Ir, the positional relationship between the subject eye and the imaging unit can be adjusted while checking a positional relationship between the light projection region and the light receiving region and a pupil in real time.

Further, at this time, fundus observation images may be simultaneously displayed on the monitor. Since the positional relationship between the subject eye and the imaging unit can be adjusted while checking a state and the like of unevenness in brightness of a fundus image, it is easy to adjust the positional relationship such that a more satisfactory fundus image can be obtained.

Further, the control unit may display the anterior eye segment observation image and the fundus observation image and may individually set an irradiation state (ON/OFF of illumination light) of illumination light of the respective light projection regions based on an operation on the input interface. Then, the set irradiation state may be reflected in imaging of the fundus observation image. Thereby, an examiner can search for an imaging condition for obtaining a satisfactory fundus image while manually changing the positional relationship between the subject eye and the imaging unit and the irradiation state of the illumination light from the respective light projection regions. In the example of FIG. 15, it is possible to switch ON/OFF of the illumination light from the light projection region corresponding to the index Ip by placing a cursor C on the index Ip on the anterior eye segment image for selection.

### Reference Signs List

1 fundus imaging device
10 imaging optical system
15a, 15b slit-shaped member
15c drive unit
100 control unit
P1, P2 light projection region
R light receiving region

## Claims

1. A fundus (Er) imaging device (1) comprising:
an imaging optical system (10) that includes light projection and reception separating means that forms a light projection region (P1, P2), through which illumination light passes onto a pupil (Ep) of a subject eye (E), and a light receiving region (R), from which fundus (Er) reflection light of the illumination light is extracted on the pupil (Ep) of the subject eye (E), at different positions, and a light receiving element (28) that receives the fundus (Er) reflection light extracted from the light receiving region (R); and
a drive unit (8) that adjusts a positional relationship between the subject eye (E) and the imaging optical system (10),
wherein the fundus (Er) imaging device (1) obtains a fundus (Er) image based on a signal from the light receiving element (28), and
the fundus (Er) imaging device (1) further comprises imaging control means (100) that enables to selectively set, as an alignment reference position being a reference for alignment between the subject eye (E) and the imaging optical system (10), any one of a first reference position which is assumed that all the light projection region (P1, P2) and the light receiving region (R) are formed inside a pupil (Ep) region of the subject eye (E), and a second reference position which is different from the first reference position and is assumed that a part of any one of the light projection region (P 1, P2) and the light receiving region (R) is formed outside the pupil (Ep) region of the subject eye (E), and that induces alignment based on an alignment deviation from the set alignment reference position,
wherein the light projection and reception separating means forms two light projection regions (P 1, P2) having different positions on a pupil (Ep) of the subject eye (E),
the imaging control means (100) sets a position, as the second reference position, at which one of the two light projection regions (P 1, P2) together with the light receiving region (R) are disposed inside a pupil (Ep) of the subject eye (E) more preferentially than a remaining one light projection region (P1, P2), **characterized in that**
the imaging control means (100) causes the illumination light to be projected to a fundus (Er) from both of the two light projection regions (P 1, P2) when the fundus (Er) image is captured in a case of inducing alignment to the first reference position, and causes the illumination light to be projected to the fundus (Er) from only one of the two light projection regions (P1, P2), which is preferentially disposed inside the pupil (Ep), when the fundus (Er) image is captured in a case of inducing alignment to the second reference position.

2. The fundus (Er) imaging device (1) according to claim 1, further comprising:
an anterior eye segment observation optical system (40) that obtains an anterior eye segment observation image,
wherein the imaging control means (100) sets the alignment reference position based on information regarding a pupil (Ep) region of the subject eye (E) acquired from the anterior eye segment observation image.

3. The fundus (Er) imaging device (1) according to claim 2,
wherein the information regarding the pupil (Ep) region includes a ratio of a part , which is disposed inside the pupil (Ep) region, of the light projection region (P1, P2) and the light receiving region (R).

4. The fundus (Er) imaging device (1) according to any one of claims 1 to 3, further comprising:
observation image obtaining means (100) that obtains a fundus (Er) observation image of the fundus (Er) image,
wherein the imaging control means (100) sets the alignment reference position based on information regarding brightness of the fundus (Er) observation image.

5. The fundus (Er) imaging device (1) according to any one of claims 1 to 4, further comprising:
an anterior eye segment observation optical system (40) that obtains an anterior eye segment observation image of the subject eye (E); and
operation input means (100) that receives an operation of an examiner and drives the drive unit (8) according to the operation to adjust the relative position,
wherein the imaging control means (100) includes display control means (100) that displays the anterior eye segment observation image on a monitor (120) and displays a guide, which guides an operation to the target position, on the anterior eye segment observation image based on the alignment reference position.

6. The fundus (Er) imaging device (1) according to any one of claims 1 to 5,
wherein the imaging control means (100) drives and controls the drive unit (8) based on an alignment deviation from the alignment reference position to induce alignment.

7. The fundus (Er) imaging device (1) according to any one of claims 1 to 6,
wherein the remaining one of the two light projection regions (P1, P2) is disposed outside the pupil (Ep) at the second reference position.

8. The fundus (Er) imaging device (1) according to any one of claims 1 to 7,
wherein a center of the light receiving region (R) matches a center of the pupil (Ep) or a center of a cornea of the subject eye (E) at the first reference position.

9. The fundus (Er) imaging device (1) according to any one of claims 1 to 8,
wherein in a case of inducing alignment to the second reference position, the imaging control means (100) increases at least one of an amount of the illumination light and a gain of the imaging element to capture the fundus (Er) image, as compared with a case of inducing alignment to the first reference position.

10. The fundus (Er) imaging device (1) according to any one of claims 1 to 9,
wherein the light projection and reception separating means forms the two light projection regions (P1, P2) and the light receiving region (R) at positions separated in a left-right direction.

11. The fundus (Er) imaging device (1) according to any one of claims 1 to 9,
wherein the light projection and reception separating means forms the two light projection regions (P1, P2) at positions vertically separated from each other, and
the imaging control means (100) sets the second reference position at which one of the two light projection regions (P1, P2) formed on a lower side is disposed inside the pupil (Ep), and projects the illumination light from the one when capturing the fundus (Er) image.

12. The fundus (Er) imaging device (1) according to any one of claims 1 to 11,
wherein the light projection and reception separating means forms the light receiving region (R) to be interposed between the two light projection regions (P1, P2) on the pupil (Ep) of the subject eye (E).

13. The fundus (Er) imaging device (1) according to any one of claims 1 to 12,
wherein the imaging optical system (10) further includes:
a slit formation unit (15a) that forms the illumination light in a slit shape on a fundus (Er) of the subject eye (E); and
a scan unit that scans the illumination light formed in the slit shape on the fundus (Er) in a direction orthogonal to a slit, and
the light projection and reception separating means forms the light projection regions (P 1, P2) at two positions separated from each other in a scan direction of the scan unit on the pupil (Ep) of the subject eye (E), and forms the light receiving region (R) to be interposed between the two light projection regions (P 1, P2) on the pupil (Ep) of the subject eye (E).

14. The fundus (Er) imaging device (1) according to any one of claims 1 to 13,
wherein the imaging control means (100) captures a first fundus (Er) image which is a fundus (Er) image based on the illumination light projected from one of the two light projection regions (P1, P2), and a second fundus (Er) image which is a fundus (Er) image based on the illumination light projected from the other of the two light projection regions (P1, P2), after alignment to the first reference position is completed, and
the fundus (Er) imaging device (1) further comprises image processing means (100) that generates a composite image with using at least two of the first fundus (Er) image and the second fundus (Er) image.

## Patentansprüche

1. Fundus (Er)-Abbildungsvorrichtung (1), umfassend:
ein optisches Abbildungssystem (10), das Lichtprojektions- und -empfangstrennmittel, die einen Lichtprojektionsbereich (P1, P2), durch den Beleuchtungslicht auf eine Pupille (Ep) eines Objektauges (E) passiert, und einen Lichtempfangsbereich (R), von dem Fundus (Er)-Reflexionslicht des Beleuchtungslichts auf die Pupille (Ep) des Objektauges (E) an unterschiedlichen Positionen extrahiert wird, und ein Lichtempfangselement (28), das das vom Lichtempfangsbereich (R) extrahierte Fundus (Er)-Reflexionslicht empfängt, aufweist; und
eine Antriebseinheit (8), die eine positionelle Beziehung zwischen dem Objektauge (E) und dem optischen Abbildungssystem (10) einstellt,
wobei die Fundus (Er)-Abbildungsvorrichtung (1) ein Fundus (Er)-Bild basierend auf einem Signal von dem Lichtempfangselement (28) empfängt, und
die Fundus (Er)-Abbildungsvorrichtung (1) ferner eine Abbildungssteuereinheit (100), die ermöglicht, als eine Ausrichtungsreferenzposition, die eine Referenz für Ausrichtung zwischen dem Objektauge (E) und dem optischen Abbildungssystem (10) ist, eine beliebige von einer ersten Referenzposition, von der angenommen ist, dass der gesamte Lichtprojektionsbereich (P1, P2) und der Lichtempfangsbereich (R) innerhalb eines Pupillen-(Ep)-Bereichs des Objektauges (E) geformt sind, und einer zweiten Referenzposition aufweistselektiv einzustellen, die sich von der ersten Referenzposition unterscheidet und davon ausgeht, dass ein Abschnitt von einem des Lichtprojektionsbereichs (P1, P2) und des Lichtempfangsbereichs (R) außerhalb des Pupillen-(Ep)-Bereichs des Objektauges (E) ausgebildet ist, und die Ausrichtung basierend auf einer Ausrichtungsabweichung von der eingestellten Ausrichtungsreferenzposition induziert,
wobei das Lichtprojektions- und -empfangstrennmittel zwei Lichtprojektionsbereiche (P1, P2) formt, die unterschiedliche Positionen auf einer Pupille (Ep) des Objektauges (E) aufweisen,
die Abbildungssteuereinheit (100) eine Position als die zweite Referenzposition festlegt, an der einer der beiden Lichtprojektionsbereiche (P1, P2) zusammen mit dem Lichtempfangsbereich (R) innerhalb einer Pupille (Ep) des Objektauges (E) bevorzugter angeordnet ist als ein verbleibender Lichtprojektionsbereich (P1, P2), **dadurch gekennzeichnet, dass**
die Abbildungssteuereinheit (100) bewirkt, dass das Beleuchtungslicht von beiden der zwei Lichtprojektionsbereiche (P1, P2) auf einen Fundus (Er) projiziert wird, wenn das Fundus (Er)-Bild in einem Fall der Induzierung von Ausrichtung auf die erste Referenzposition erfasst wird, und bewirkt, dass das Beleuchtungslicht nur von einem der beiden Lichtprojektionsbereiche (P1, P2), der vorzugsweise innerhalb der Pupille (Ep) angeordnet ist, auf den Fundus (Er) projiziert wird, wenn das Fundus (Er)-Bild in einem Fall aufgenommen wird, in dem Ausrichtung auf die zweite Referenzposition induziert wird.

2. Fundus (Er)-Abbildungsvorrichtung (1) nach Anspruch 1, ferner umfassend:
ein optisches System (40) zum Beobachten eines vorderen Augensegments, das ein Bild zum Beobachten des vorderen Augensegments aufweist,
wobei die Abbildungssteuereinheit (100) die Ausrichtungsreferenzposition basierend auf Informationen bezüglich eines Pupillen (Ep)-Bereichs des Objektauges (E) einstellt, die von dem Bild zum Beobachten des vorderen Augensegments erfasst werden.

3. Fundus (Er)-Abbildungsvorrichtung (1) nach Anspruch 2,
wobei die Informationen bezüglich des Pupillen-(Ep)-Bereichs ein Verhältnis eines Abschnitts, der innerhalb des Pupillen-(Ep)-Bereichs angeordnet ist, des Lichtprojektionsabschnitts (P1, P2) und des Lichtempfangsbereichs (R) aufweisen.

4. Fundus (Er)-Abbildungsvorrichtung (1) nach einem der Ansprüche 1 bis 3, ferner umfassend:
Beobachtungsbild-Erhaltungsmittel (100), das ein Fundus (Er)-Beobachtungsbild des Fundus (Er)-Bildes aufweist,
wobei die Abbildungssteuereinheit (100) die Ausrichtungsreferenzposition basierend auf Informationen bezüglich Helligkeit des Fundus (Er)-Beobachtungsbildes einstellt.

5. Fundus (Er)-Abbildungsvorrichtung (1) nach einem der Ansprüche 1 bis 4, ferner umfassend:
ein optisches System (40) zum Beobachten eines vorderen Augensegments, das ein Beobachtungsbild des vorderen Augensegments des Objektauges (E) aufweist; und
Betätigungseingabemittel (100), das eine Betätigung eines Prüfers empfängt und die Antriebseinheit (8) gemäß der Betätigung antreibt, um die relative Position einzustellen,
wobei das Abbildungssteuermittel (100) Anzeigesteuermittel (100) aufweist, das das Bild zum Beobachten eines vorderen Augensegments auf einem Monitor (120) anzeigt und eine Führung, die eine Betätigung zu der Zielposition führt, auf dem Bild zum Beobachten des vorderen Augensegments basierend auf der Ausrichtungsreferenzposition anzeigt.

6. Fundus (Er)-Abbildungsvorrichtung (1) nach einem der Ansprüche 1 bis 5,
wobei das Abbildungssteuermittel (100) die Antriebseinheit (8) basierend auf einer Ausrichtungsabweichung von der Ausrichtungsreferenzposition antreibt und steuert, um Ausrichtung zu induzieren.

7. Fundus (Er)-Abbildungsvorrichtung (1) nach einem der Ansprüche 1 bis 6,
wobei der verbleibende der beiden Lichtprojektionsabschnitte (P1, P2) an der zweiten Referenzposition außerhalb der Pupille (Ep) angeordnet ist.

8. Fundus (Er)-Abbildungsvorrichtung (1) nach einem der Ansprüche 1 bis 7,
wobei ein Zentrum des Lichtempfangsabschnitts (R) mit einem Zentrum der Pupille (Ep) oder einem Zentrum einer Cornea des Objektauges (E) an der ersten Referenzposition übereinstimmt.

9. Fundus (Er)-Abbildungsvorrichtung (1) nach einem der Ansprüche 1 bis 8,
wobei in einem Fall, in dem Ausrichtung auf die zweite Referenzposition induziert wird, das Abbildungssteuermittel (100) zumindest eine einer Menge des Beleuchtungslichts und eine Zunahme des Abbildungselements erhöht, um das Fundus (Er)-Bild im Vergleich zu einem Fall, in dem Ausrichtung auf die erste Referenzposition induziert wird, aufzunehmen.

10. Fundus (Er)-Abbildungsvorrichtung (1) nach einem der Ansprüche 1 bis 9,
wobei das Lichtprojektions- und -empfangstrennmittel die beiden Lichtprojektionsabschnitte (P1, P2) und den Lichtempfangsabschnitt (R) an in einer Links-Rechts-Richtung getrennten Positionen formt.

11. Fundus (Er)-Abbildungsvorrichtung (1) nach einem der Ansprüche 1 bis 9,
wobei das Lichtprojektions- und -empfangstrennmittel die beiden Lichtprojektionsbereiche (P1, P2) an vertikal voneinander getrennten Positionen formt, und
das Abbildungssteuermittel (100) die zweite Referenzposition einstellt, an der einer der beiden auf einer unteren Seite geformten Lichtprojektionsbereiche (P1, P2) innerhalb der Pupille (Ep) angeordnet ist, und das Beleuchtungslicht von diesem projizieren, wenn das Fundus (Er)-Bild aufgenommen wird.

12. Fundus (Er)-Abbildungsvorrichtung (1) nach einem der Ansprüche 1 bis 11,
wobei das Lichtprojektions- und -empfangstrennmittel den zwischen den zwei Lichtprojektionsbereichen (P1, P2) auf der Pupille (Ep) des Objektauges (E) angeordneten Lichtempfangsbereich (R) formt.

13. Fundus (Er)-Abbildungsvorrichtung (1) nach einem der Ansprüche 1 bis 12,
wobei das optische Abbildungssystem (10) ferner aufweist:
eine Schlitzformungseinheit (15a), die das Beleuchtungslicht in einer Schlitzform auf einen Fundus (Er) des Objektauges (E) formt; und
eine Scaneinheit, die das Beleuchtungslicht, das in der Schlitzform auf den Fundus (Er) geformt ist, in einer Richtung orthogonal zu einem Schlitz scannt, und
das Lichtprojektions- und -empfangstrennmittel die Lichtprojektionsbereiche (P1, P2) an zwei in einer Scanrichtung der Scaneinheit voneinander getrennten Positionen auf die Pupille (Ep) des Objektauges (E) formt und den Lichtempfangsbereich (R) formt, der zwischen den beiden Lichtprojektionsbereichen (P1, P2) auf die Pupille (Ep) des Objektauges (E) anzuordnen ist.

14. Fundus (Er)-Abbildungsvorrichtung (1) nach einem der Ansprüche 1 bis 13,
wobei das Abbildungssteuermittel (100) ein erstes Fundus (Er)-Bild, das ein Fundus (Er)-Bild basierend auf dem Beleuchtungslicht ist, das von einem der beiden Lichtprojektionsbereiche (P1, P2) projiziert wird, und ein zweites Fundus (Er)-Bild, das ein Fundus (Er)-Bild basierend auf dem Beleuchtungslicht ist, das von dem anderen der zwei Lichtprojektionsbereiche (P1, P2) projiziert wird, aufnimmt, nachdem Ausrichtung auf die erste Referenzposition abgeschlossen ist, und
die Fundus (Er)-Abbildungsvorrichtung (1) ferner Bildverarbeitungsmittel (100) aufweist, das ein zusammengesetztes Bild unter Verwendung von mindestens zweien von dem ersten Fundus (Er)-Bild und dem zweiten Fundus (Er)-Bild erzeugt.

## Revendications

1. Dispositif d'imagerie de fond d'oeil (Er) (1) comprenant :
- un système optique d'imagerie (10) qui comprend un moyen de séparation de projection et de réception de lumière qui forme une région de projection de lumière (P1, P2), à travers laquelle une lumière d'éclairage passe sur une pupille (Ep) d'un oeil du sujet (E), et une région de réception de lumière (R), à partir de laquelle une lumière de réflexion de fond d'oeil (Er) de la lumière d'éclairage est extraite sur la pupille (Ep) de l'oeil du sujet (E), à différentes positions, et un élément de réception de lumière (28) qui reçoit la lumière de réflexion de fond d'oeil (Er) extraite de la région de réception de lumière (R) ; et
- une unité d'entraînement (8) qui ajuste une relation de position entre l'oeil du sujet (E) et le système optique d'imagerie (10),
dans lequel le dispositif d'imagerie de fond d'oeil (Er) (1) obtient une image de fond d'oeil (Er) sur la base d'un signal provenant de l'élément de réception de lumière (28), et
le dispositif d'imagerie de fond d'oeil (Er) (1) comprend en outre un moyen de commande d'imagerie (100) qui permet de définir sélectivement, en tant que position de référence d'alignement qui est une référence pour l'alignement entre l'oeil du sujet (E) et le système optique d'imagerie (10), l'une quelconque d'une première position de référence qui suppose que toute la région de projection de lumière (P1, P2) et la région de réception de lumière (R) sont formées à l'intérieur d'une région de pupille (Ep) de l'oeil du sujet (E), et d'une seconde position de référence qui est différente de la première position de référence et suppose qu'une partie de l'une quelconque de la région de projection de lumière (P1, P2) et de la région de réception de lumière (R) est formée à l'extérieur de la région de pupille (Ep) de l'oeil du sujet (E), et qui déclenche un alignement sur la base d'un écart d'alignement par rapport à la position de référence d'alignement définie,
dans lequel le moyen de séparation de projection et de réception de lumière forme deux régions de projection de lumière (PI, P2) ayant des positions différentes sur une pupille (Ep) de l'oeil du sujet (E),
le moyen de commande d'imagerie (100) définit une position, comme étant la seconde position de référence, à laquelle l'une des deux régions de projection de lumière (P1, P2) ainsi que la région de réception de lumière (R) sont disposées à l'intérieur d'une pupille (Ep) de l'oeil du sujet (E) plus préférentiellement qu'une région de projection de lumière restante (P1, P2),
**caractérisé en ce que** le moyen de commande d'imagerie (100) amène la lumière d'éclairage à être projetée sur un fond d'oeil (Er) à partir des deux régions de projection de lumière (P1, P2) lorsque l'image de fond d'oeil (Er) est capturée dans le cas de déclenchement d'un alignement sur la première position de référence, et amène la lumière d'éclairage à être projetée sur le fond d'oeil (Er) à partir d'une seule des deux régions de projection de lumière (P1, P2), qui est préférentiellement disposée à l'intérieur de la pupille (Ep), lorsque l'image de fond d'oeil (Er) est capturée dans le cas de déclenchement d'un alignement sur la seconde position de référence.

2. Dispositif d'imagerie de fond d'oeil (Er) (1) selon la revendication 1, comprenant en outre un système optique d'observation de segment antérieur de l'oeil (40) qui obtient une image d'observation de segment antérieur de l'oeil,
dans lequel le moyen de commande d'imagerie (100) définit la position de référence d'alignement sur la base d'informations concernant une région de pupille (Ep) de l'oeil du sujet (E) acquises à partir de l'image d'observation de segment antérieur de l'oeil.

3. Dispositif d'imagerie de fond d'oeil (Er) (1) selon la revendication 2, dans lequel les informations concernant la région de pupille (Ep) comprennent un rapport d'une partie, qui est disposée à l'intérieur de la région de pupille (Ep), de la région de projection de lumière (P1, P2) et de la région de réception de lumière (R).

4. Dispositif d'imagerie de fond d'oeil (Er) (1) selon l'une quelconque des revendications 1 à 3, comprenant en outre un moyen d'obtention d'image d'observation (100) qui obtient une image d'observation de fond d'oeil (Er) de l'image de fond d'oeil (Er),
dans lequel le moyen de commande d'imagerie (100) définit la position de référence d'alignement sur la base d'informations concernant la luminosité de l'image d'observation de fond d'oeil (Er).

5. Dispositif d'imagerie de fond d'oeil (Er) (1) selon l'une quelconque des revendications 1 à 4, comprenant en outre :
- un système optique d'observation de segment antérieur de l'oeil (40) qui obtient une image d'observation de segment antérieur de l'oeil de l'oeil du sujet (E) ; et
- un moyen d'entrée d'opération (100) qui reçoit une opération d'un examinateur et entraîne l'unité d'entraînement (8) selon l'opération pour ajuster la position relative,
dans lequel le moyen de commande d'imagerie (100) comprend un moyen de commande d'affichage (100) qui affiche l'image d'observation de segment antérieur de l'oeil sur un moniteur (120) et affiche un guide, qui guide une opération vers la position cible, sur l'image d'observation de segment antérieur de l'oeil sur la base de la position de référence d'alignement.

6. Dispositif d'imagerie de fond d'oeil (Er) (1) selon l'une quelconque des revendications 1 à 5, dans lequel le moyen de commande d'imagerie (100) entraîne et commande l'unité d'entraînement (8) sur la base d'un écart d'alignement par rapport à la position de référence d'alignement pour déclencher un alignement.

7. Dispositif d'imagerie de fond d'oeil (Er) (1) selon l'une quelconque des revendications 1 à 6, dans lequel la région restante des deux régions de projection de lumière (P1, P2) est disposée à l'extérieur de la pupille (Ep) à la seconde position de référence.

8. Dispositif d'imagerie de fond d'oeil (Er) (1) selon l'une quelconque des revendications 1 à 7, dans lequel un centre de la région de réception de lumière (R) correspond à un centre de la pupille (Ep) ou à un centre d'une cornée de l'oeil du sujet (E) à la première position de référence.

9. Dispositif d'imagerie de fond d'oeil (Er) (1) selon l'une quelconque des revendications 1 à 8, dans lequel, dans le cas de déclenchement d'un alignement sur la seconde position de référence, le moyen de commande d'imagerie (100) augmente au moins l'un d'une quantité de la lumière d'éclairage et d'un gain de l'élément d'imagerie pour capturer l'image de fond d'oeil (Er), par rapport au cas de déclenchement d'un alignement sur la première position de référence.

10. Dispositif d'imagerie de fond d'oeil (Er) (1) selon l'une quelconque des revendications 1 à 9, dans lequel le moyen de séparation de projection et de réception de lumière forme les deux régions de projection de lumière (P1, P2) et la région de réception de lumière (R) à des positions séparées dans une direction gauche-droite.

11. Dispositif d'imagerie de fond d'oeil (Er) (1) selon l'une quelconque des revendications 1 à 9, dans lequel le moyen de séparation de projection et de réception de lumière forme les deux régions de projection de lumière (P1, P2) à des positions verticalement séparées l'une de l'autre, et
le moyen de commande d'imagerie (100) définit la seconde position de référence à laquelle l'une des deux régions de projection de lumière (P1, P2) formée sur un côté inférieur est disposée à l'intérieur de la pupille (Ep), et projette la lumière d'éclairage depuis celle-ci lors de la capture de l'image de fond d'oeil (Er).

12. Dispositif d'imagerie de fond d'oeil (Er) (1) selon l'une quelconque des revendications 1 à 11, dans lequel le moyen de séparation de projection et de réception de lumière forme la région de réception de lumière (R) à interposer entre les deux régions de projection de lumière (P1, P2) sur la pupille (Ep) de l'oeil du sujet (E).

13. Dispositif d'imagerie de fond d'oeil (Er) (1) selon l'une quelconque des revendications 1 à 12, dans lequel le système optique d'imagerie (10) comprend en outre :
- une unité de formation de fente (15a) qui forme la lumière d'éclairage sous forme de fente sur un fond d'oeil (Er) de l'oeil du sujet (E) ; et
- une unité de balayage qui balaie la lumière d'éclairage formée sous la forme de fente sur le fond d'oeil (Er) dans une direction orthogonale à une fente,
et
le moyen de séparation de projection et de réception de lumière forme les régions de projection de lumière (P1, P2) à deux positions séparées l'une de l'autre dans une direction de balayage de l'unité de balayage sur la pupille (Ep) de l'oeil du sujet (E), et forme la région de réception de lumière (R) à interposer entre les deux régions de projection de lumière (P1, P2) sur la pupille (Ep) de l'oeil du sujet (E).

14. Dispositif d'imagerie de fond d'oeil (Er) (1) selon l'une quelconque des revendications 1 à 13, dans lequel le moyen de commande d'imagerie (100) capture une première image de fond d'oeil (Er) qui est une image de fond d'oeil (Er) basée sur la lumière d'éclairage projetée depuis l'une des deux régions de projection de lumière (P1, P2), et une seconde image de fond d'oeil (Er) qui est une image de fond d'oeil (Er) basée sur la lumière d'éclairage projetée depuis l'autre des deux régions de projection de lumière (P1, P2), après que l'alignement sur la première position de référence est terminé, et
le dispositif d'imagerie de fond d'oeil (Er) (1) comprend en outre un moyen de traitement d'image (100) qui génère une image composite en utilisant au moins deux de la première image de fond d'oeil (Er) et de la seconde image de fond d'oeil (Er).
